# EUROPEAN PATENT APPLICATION

(11) **EP 4 563 125 A2**
(43) Date of publication of application: **04.06.2025**
(21) Application number: 25157058.6
(22) Date of filing: 05.01.2021
(51) Int. Cl.: A61F 2/82

(54) **PROSTHETIC HEART VALVE LEAFLET ASSEMBLIES AND METHODS**

(30) Priority: 10.01.2020 US 202062959433 P
(62) Divisional of application: 23191760.0
(71) Applicant: Edwards Lifesciences Corporation, Irvine, CA 92614 (US)
(72) Inventor: NIR, Noam, 30889 Caesarea (IL); BUKIN, Michael, 30889 Caesarea (IL); SHERMAN, Elena, 30889 Caesarea (IL)
(74) Representative: Venner, Julia Ann

(57) **Abstract**

An assembly and associated method for a commissure of a prosthetic heart valve is disclosed. As one example, a method includes forming each of a plurality of commissures with a plurality of leaflets by pairing a first commissure tab of a first leaflet with an adjacent, second commissure tab of a second leaflet, folding a support strip over an inner reinforcing element, positioning the folded support strip between the first and second commissure tabs, and attaching first and second outer reinforcing elements to the first and second commissure tabs, respectively, via stitching, the stitching extending over at least a portion of the first reinforcing element opposite a fold region of the support strip, and, for each commissure, securing end portions of the support strip to a respective support portion of a frame of the prosthetic heart valve.

## Description

### CROSS REFERENCE TO RELATED APPLICATIONS

This application claims the benefit of U.S. Provisional Application 62/959,433 filed on January 10, 2020, which is incorporated by reference herein in its entirety.

### FIELD

The present disclosure relates to prosthetic heart valves, and to methods and assemblies for forming commissures associated with leaflets of such prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery device so that the prosthetic valve can self-expand to its functional size.

Prosthetic valves that rely on a mechanical actuator for expansion can be referred to as "mechanically expandable" prosthetic heart valves. The actuator typically takes the form of pull cables, sutures, wires and/or shafts that are configured to transmit expansion forces from a handle of the delivery apparatus to the prosthetic valve. Most expandable, transcatheter heart valves comprise a cylindrical metal frame or stent and prosthetic leaflets mounted inside the frame. The leaflets may be attached to the frame at commissure tab of the leaflets. However, in some configurations, forces experienced during operation of the prosthetic valve and/or other conditions may be concentrated at the commissure tabs, which may compromise the structure of the leaflets and/or cause the leaflets to detach from the frame. Furthermore, the attachment of the commissure tabs to the frame in such configurations may require delicate assembly skills to ensure proper attachment and reduce damage to the leaflets.

Accordingly, a need exists for improved prosthetic heart valve leaflet assemblies and methods for assembling the leaflet assemblies to a frame of the prosthetic heart valve.

### SUMMARY

Described herein are embodiments of methods for assembling a prosthetic heart valve, methods of assembling a commissure of a prosthetic valve, and a prosthetic heart valve including a plurality of commissures. In some embodiments, the commissures may be formed by coupling a pair of adjacent commissure tabs of adjacent leaflets of the prosthetic heart valve. The commissures may include a support strip folded over an inner reinforcing element and positioned between the adjacent commissure tabs, and an outer reinforcing member including first and second outer reinforcing elements that are respectively positioned on outer surfaces of the adjacent commissure tabs and attached thereto via stitching, such that the inner reinforcing element is positioned between the stitching and a fold region of the support strip.

In one representative embodiment, a method of assembling a prosthetic heart valve including a plurality of leaflets can include: forming a plurality of commissures with the plurality of leaflets, wherein each commissure is formed by pairing a first commissure tab of a first leaflet with an adjacent, second commissure tab of a second leaflet, folding a support strip over an inner reinforcing element, positioning the folded support strip between the first commissure tab and the second commissure tab, attaching first and second outer reinforcing elements to the first and second commissure tabs, respectively, via stitching that extends through the first outer reinforcing element, the first leaflet, the folded support strip, the second leaflet, and the second outer reinforcing element, the stitching extending over at least a portion of the first reinforcing element opposite a fold region of the support strip, and, for each commissure, securing end portions of the support strip to a respective support portion of a frame of the prosthetic heart valve.

In another representative embodiment, a prosthetic heart valve can include: an annular frame comprising a plurality of commissure support portions, and a plurality of leaflets, each leaflet having a commissure tab that is coupled to an adjacent commissure tab of another leaflet via a folded support strip to form a commissure of an associated commissure tab pair, wherein, for each commissure tab pair: the folded support strip is disposed between respective inner surfaces of the commissure tabs in the commissure tab pair, an inner reinforcing element is disposed in a fold region of the folded support strip, first and second outer reinforcing elements are positioned adjacent respective outer surfaces of the commissure tabs in the commissure tab pair, a reinforcing suture passes through, in order: the first outer reinforcing element, a first commissure tab of the commissure tab pair, a first section of the folded support strip, a second section of the folded support strip, a second commissure tab of the commissure tab pair, and a second outer reinforcing element, and the inner reinforcing element is positioned between the fold region of the folded support strip and the reinforcing suture, and wherein each commissure is secured to a corresponding commissure support portion of the plurality of commissure support portions.

In yet another representative embodiment, a method of assembling a commissure for a leaflet pair of a prosthetic valve can include: folding a support strip over an inner reinforcing element to form a first folded portion of the support strip extending from a fold region of the support strip to a first end portion and a second folded portion of the support strip extending from the fold region of the support strip to a second end portion opposite the first end portion, positioning the folded support strip between inner surfaces of adjacent ends of a first commissure tab of a first leaflet of the leaflet pair and a second commissure tab of a second leaflet of the leaflet pair, folding an outer reinforcing member into a U-shape to form a first outer reinforcing element and a second outer reinforcing element, placing the first outer reinforcing element against an outer surface of the first commissure tab and the second outer reinforcing element against an outer surface of the second commissure, forming, via a suture, one or more stitches that pass through, in order, the first outer reinforcing element, the first commissure tab, the first folded portion of the support strip, the second folded portion of the support strip, the second commissure tab, and the second outer reinforcing element, so that the inner reinforcing element is positioned between the fold region of the support strip and the suture, and coupling the first end portion of the folded support strip to the second end portion of the folded support strip around a support portion of a frame of the prosthetic valve to secure the commissure to the support portion of the frame.

In yet another representative embodiment, a method of assembling a prosthetic heart valve comprising a plurality of leaflets includes forming a plurality of commissures with the plurality of leaflets, wherein each commissure is formed by pairing a first commissure tab of a first leaflet with an adjacent, second commissure tab of a second leaflet, folding a first portion of a support strip over a first edge reinforcing element, folding a second portion of the support strip over a second edge reinforcing element, coupling an end portion of the first portion of the support strip to an end portion of the second portion of the support strip to form a reinforced region of the support strip, positioning the support strip along respective inner surfaces of the first commissure tab and the second commissure tab, coupling first and second outer reinforcing elements to the first and second commissure tabs, respectively, via stitching that extends through the first outer reinforcing element, the first commissure tab, the folded support strip, the second commissure tab, and the second outer reinforcing element, the stitching extending over the reinforced region of the support strip, and for each commissure, securing the support strip to a respective support portion of a frame of the prosthetic heart valve.

In yet another representative embodiment, a method of assembling a prosthetic heart valve comprising a plurality of leaflets includes forming a plurality of commissures with the plurality of leaflets, wherein each commissure is formed by pairing a first commissure tab of a first leaflet with an adjacent, second commissure tab of a second leaflet, the first commissure tab comprising a first extended tab and the second commissure tab comprising a second extended tab, folding the first and second extended tabs over first and second portions of a primary reinforcing element, respectively, placing first and second portions of a secondary reinforcing element on respective outer surfaces of the folded first and second extended tabs, and coupling end portions of the first and second portions of the secondary reinforcing element to one another, and, for each commissure, coupling a fold region of the first and second extended tabs to a support material and securing the support material and/or the first and second extended tabs to an associated support structure of a frame of the prosthetic heart valve.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of an exemplary embodiment of a prosthetic heart valve.
FIG. 2 illustrates a first stage of assembling a commissure of a prosthetic valve, including attaching an inner reinforcing element to a support strip.
FIG. 3 illustrates a second stage of assembling the commissure, including folding the support strip widthwise over the inner reinforcing element.
FIG. 4 illustrates a third stage of assembling the commissure, including placing the folded support strip between adjacent commissure tabs of adjacent leaflets and placing first and second outer reinforcing elements of an outer reinforcing member along outer surfaces of the commissure tabs.
FIG. 5 illustrates a fourth stage of assembling the commissure, including attaching the first and second outer reinforcing elements to the adjacent commissure tabs and the folded support strip.
FIG. 6 is a cross-sectional view of the commissure after attaching the first and second outer reinforcing elements to the adjacent commissure tabs and the folded support strip.
FIG. 7 illustrates one example of a fifth stage of assembling the commissure, including attaching sutures to opposing ends of the folded support strip.
FIG. 8 illustrates an optional additional stage of assembling the commissure, including attaching the folded support strip to the adjacent commissure tabs via supplemental sutures.
FIG. 9 illustrates one example of a sixth stage of assembling the commissure, including coupling the opposing end portions of the folded support strip with one another around a support post of a frame of the prosthetic valve via the sutures attached to the end portions of the folded support strip in order to secure the commissure to the support post.
FIG. 10 is another example of the fifth stage of assembling the commissure, including respectively folding opposing end portions of the folded support strip over corresponding end portions of the adjacent commissure tabs and attaching the folded ends of the support strip to the corresponding end portions of the adjacent commissure tabs via respective sutures.
FIG. 11 is another example of the sixth stage of assembling the commissure, including coupling the respective sutures to one another around the support post of the frame of the prosthetic valve in order to secure the commissure to the support post.
FIG. 12 is another example of the fifth and sixth stages of assembling the commissure, including attaching the folded support strip to ends of the adjacent commissure tabs via respective sutures, wrapping opposing end portions of the folded support strip around the support post of the frame of the prosthetic valve, and attaching the opposing end portions of the folded support strip to one another via a single suture.
FIG. 13 is a flow chart of an example method of assembling a commissure of a prosthetic valve using an inner reinforcing member.
FIG. 14 is another example of assembling a commissure having a reinforced zone formed by folded end portions of a support strip and edge reinforcing members.
FIG. 15 is a detailed view of a portion of the support strip of FIG. 14 folded around an edge reinforcing member.
FIGS. 16A and 16B are detailed views of another example of a portion of a support strip that may be used for the commissure of FIG. 14 where portions of the support strip are folded over itself to reinforce edge fold regions of the support strip.
FIG. 17 is a flow chart of an example method for assembling a commissure of a prosthetic valve using edge reinforcing members.
FIG. 18 is another example of assembling a commissure of a prosthetic valve including folding extended commissure tabs of leaflets of the prosthetic valve over an inner reinforcing member.
FIG. 19 is a cross-sectional view of the example of FIG. 18.
FIG. 20A is a first stage of an example of assembling a commissure of a prosthetic valve including extended commissure tabs.
FIG. 20B is a cross-sectional view of the example portion of the prosthetic valve shown in FIG. 20A.
FIG. 20C is a second stage of the example of assembling the commissure of FIG. 20A.
FIG. 21 is a flow chart of an example method for assembling a commissure of a prosthetic valve using extended commissure tabs of leaflets of the prosthetic valve.
FIG. 22 is a side elevation view of a delivery apparatus for a prosthetic heart valve, according to one example.

### DETAILED DESCRIPTION

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein, with reference to the prosthetic heart valve and the transcatheter delivery system, "proximal" refers to a position, direction, or portion of a component that is closer to the user and a handle of the delivery system that is outside the patient, while "distal" refers to a position, direction, or portion of a component that is further away from the user and the handle and closer to the implantation site. The terms "longitudinal" and "axial" refer to an axis extending in the proximal and distal directions, unless otherwise expressly defined.

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

### Examples of the Disclosed Technology

Described herein are examples of prosthetic heart valves, commissures for prosthetic valves, and methods for assembling commissures of prosthetic valves. The prosthetic heart valves may include a frame and a plurality of leaflets attached to the frame via commissures formed by joining pairs of adjacent ends of the leaflets. The formation of the commissures may include attaching a support strip folded over a first reinforcing member to the leaflets and attaching a second reinforcing member over outer surfaces of the leaflets. Opposing ends of the support strip may be joined together, directly or indirectly (e.g., via one or more sutures), and the opposing ends of the support strip and/or sutures that join the opposing ends of the support strip may be wrapped around a corresponding commissure support portion of the frame of the prosthetic valve. In this way, forces experienced by the leaflets during radial expansion and compression of the frame and/or as the leaflets open and closed during operation of the prosthetic valve may be at least partially absorbed by the support strip, reducing the stresses exerted on the leaflets. As the support strip may be made of more robust material than the leaflets, the overall strength of the commissure may be increased relative to other configurations. The use of reinforcing members both inside the folded support strip and outside the adjacent leaflets provide additional reinforcing strength for the commissure by providing a further deflection of stresses away from the potentially relatively delicate material of the leaflets.

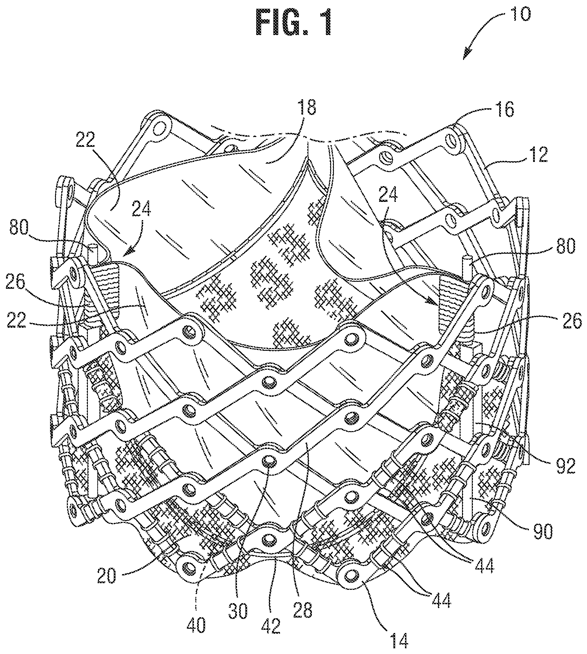

FIG. 1 shows an exemplary prosthetic heart valve 10, according to one embodiment. The prosthetic heart valve 10 can be radially compressible and expandable between a radially compressed configuration for delivery into a patient and a radially expanded configuration. In particular embodiments, the prosthetic heart valve 10 can be implanted within the native aortic valve, although it also can be implanted at other locations in the heart, including within the native mitral valve, the native pulmonary valve, and the native tricuspid valve. The prosthetic heart valve 10 can include an annular stent or frame 12 having a first end 14 and a second end 16.

In the depicted embodiment, the first end 14 is an inflow end and the second end 16 is an outflow end. The outflow end 16 can be coupled to a delivery apparatus for delivering and implanting the prosthetic heart valve within the native aortic valve is a transfemoral, retrograde delivery approach. Thus, in the delivery configuration of the prosthetic heart valve, the outflow end 16 is the proximal-most end of the prosthetic valve. In other embodiments, the inflow end 14 can be coupled to the delivery apparatus, depending on the particular native valve being replaced and the delivery technique that is used (e.g., trans-septal, transapical, etc.). For example, the inflow end 14 can be coupled to the delivery apparatus (and therefore is the proximal-most end of the prosthetic heart valve in the delivery configuration) when delivering the prosthetic heart valve to the native mitral valve via a trans-septal delivery approach.

The frame 12 can be made of any of various suitable materials, such as stainless steel, a cobalt chromium alloy, or a nickel titanium alloy ("NiTi"), for example Nitinol. Referring again to FIG. 1, as shown, the frame 12 can include a plurality of interconnected struts 28 arranged in a lattice-type pattern. The struts 28 are shown as positioned diagonally, or offset at an angle relative to, and radially offset from, a longitudinal axis of the prosthetic heart valve 10 when the prosthetic heart valve 10 is in the expanded configuration. In other implementations, the struts 28 can be offset by a different amount than depicted in FIG. 1, or some or all of the struts 28 can be positioned parallel to the longitudinal axis of the prosthetic heart valve 10.

In the illustrated embodiment, the struts 28 are pivotably coupled to one another at one or more pivot joints along the length of each strut. For example, in the illustrated configuration, each of the struts 28 can be formed with apertures at opposing ends of the strut and apertures spaced along the length of the strut. Respective hinges can be formed at the locations where struts 28 overlap each other via fasteners or pivot members, such as rivets or pins 30 that extend through the apertures. The hinges can allow the struts 28 to pivot relative to one another as the frame 12 is radially expanded or compressed, such as during assembly, preparation, or implantation of the prosthetic heart valve 10.

In some embodiments, the frame 12 can be constructed by forming individual components (e.g., the struts and fasteners of the frame) and then mechanically assembling and connecting the individual components together. In other embodiments, the struts 28 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube). Further details regarding the construction of the frame and the prosthetic heart valve are described in U.S. Patent Application Publication Nos. 2018/0153689, 2018/0344456, and 2019/0060057, all of which are incorporated herein by reference.

The prosthetic heart valve 10 can also include a valvular structure 18 which is coupled to the frame 12 and configured to regulate the flow of blood through the prosthetic heart valve 10 from the inflow end 14 to the outflow end 16. The prosthetic heart valve 10 can further include a plurality of actuators 80 mounted to and equally spaced around the inner surface of the frame 12. The actuators are configured to apply expansion and compression to the frame for radially expanding and compressing the prosthetic valve.

In the illustrated embodiment, the actuators 80 are linear actuators, each of which comprises an inner member, or piston, 90 and an outer member, or cylinder, 92. The inner member 90 is pivotably coupled to a junction of the frame, such as at the first end 14, while the outer member 92 is pivotably coupled to another junction of the frame closer to the second end 16. Moving the inner member 90 proximally relative to the outer member 92 and/or moving the outer member 92 distally relative to the inner member 90 is effective to radially expand the prosthetic valve. Conversely, moving the inner member 90 distally relative to the outer member 92 and/or moving the outer member 92 proximally relative to the inner member 90 is effective to radially compress the prosthetic valve. The actuators 80 can include locking mechanisms that are configured to retain the prosthetic valve in an expanded state inside the patient's body.

In some embodiments, each of the actuators 80 can be configured to form a releasable connection with one or more respective actuators of a delivery apparatus of a transcatheter delivery system. The actuators of the delivery apparatus can transmit forces from a handle of the delivery apparatus to the actuators 80 for expanding or compressing the prosthetic valve. Further details of the actuators, locking mechanisms and delivery apparatuses for actuating the actuators can be found in U.S. Patent Application Publication Nos. 2018/0153689, 2019/0060057 and 2018/0325665, each of which is incorporated herein by reference in its entirety. Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

In some embodiments, each of the actuators 80 can be used to support a respective commissure 24 (described below). As such, the actuators 80 can include commissure support portions for supporting and attaching commissures 24 of the valvular structure 18 to the frame 12, as described further herein.

The valvular structure 18 can include, for example, a leaflet assembly comprising one or more leaflets 22 (three leaflets 22 in the illustrated embodiment) made of a flexible material. The leaflets 22 of the leaflet assembly can be made from in whole or part, biological material, bio-compatible synthetic materials, or other such materials. Suitable biological material can include, for example, bovine pericardium (or pericardium from other sources). The leaflets 22 can be arranged to form commissures 24, which can be, for example, mounted to commissure support portions of respective actuators 80. Further details regarding transcatheter prosthetic heart valves, including the manner in which the valvular structure can be coupled to the frame 12 of the prosthetic heart valve 10, can be found, for example, in U.S. Patent Nos. 6,730,118, 7,393,360, 7,510,575, 7,993,394, and 8,652,202, and U.S. Patent Application Publication No. 2018/0325665, all of which are incorporated herein by reference in their entireties.

In some embodiments, as shown in FIG. 1, the commissures 24 can be mounted (e.g., sutured) directly to commissure support portions of the actuators 80 of the frame 12 via commissure attachments 26, which can be strips of fabric. As one example, each commissure attachment 26 may be wrapped around a corresponding actuator 80 and a pair of adjacent commissure tabs of adjacent leaflets and secured to the commissure tabs and the actuator via one or more stitches extending through the commissure attachment and the pair of the commissure tabs. In other embodiments, the commissures 24 can be mounted to support struts or posts of the frame that are separate from the actuators 80.

The prosthetic heart valve 10 can also include one or more skirts or sealing members. For example, as shown in FIG. 1, the prosthetic heart valve 10 can include an inner skirt 20 mounted on the inner surface of the frame 12. As shown in FIG. 1, the inner skirt 20 is a circumferential inner skirt that spans an entire circumference of the inner surface of the frame 12. The inner skirt 20 can function as a sealing member to prevent or decrease paravalvular leakage (e.g., when the valve is placed at the implantation site) and as an attachment surface to anchor the leaflets 22 to the frame 12.

For example, as shown, a cusp edge portion 40 of each leaflet 22 (the inflow edge portion) can be secured to the inner skirt 20 with stitching 42 (referred to as a "scallop line"). The upper and lower edge portions of the inner skirt 20 can be secured to the frame with suture loops 44 that extending through the inner skirt and around adjacent struts 28 of the frame. In this manner, the cusp edge portions of the leaflets are supported by the inner skirt 20 and the commissures are supported by actuators 80.

The prosthetic heart valve 10 can also include an outer skirt mounted on the outer surface of the frame 12 (not shown in FIG. 1). The outer skirt can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials (e.g., PET) or natural tissue (e.g., pericardial tissue). The inner and outer skirts can be mounted to the frame using sutures, an adhesive, welding, and/or other means for attaching the skirts to the frame.

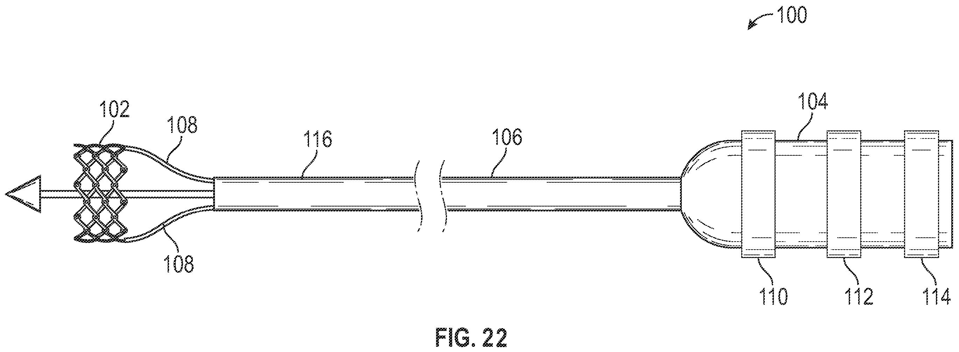

FIG. 22 illustrates a delivery apparatus 100, according to one embodiment, adapted to deliver a prosthetic heart valve 102, such as the illustrated prosthetic heart valve 10, described above with respect to FIG. 1. The prosthetic valve 102 can be releasably coupled to the delivery apparatus 100. It should be understood that the delivery apparatus 100 and other delivery apparatuses disclosed herein can be used to implant prosthetic devices other than prosthetic valves, such as stents or grafts.

The delivery apparatus 100 in the illustrated embodiment generally includes a handle 104, a first elongated shaft 106 (which comprises an outer shaft in the illustrated embodiment) extending distally from the handle 104, at least one actuator assembly 108 extending distally through the outer shaft 106. The at least one actuator assembly 108 can be configured to radially expand and/or radially collapse the prosthetic valve 102 when actuated.

Though the illustrated embodiment shows two actuator assemblies 108 for purposes of illustration, it should be understood that one actuator 108 can be provided for each actuator on the prosthetic valve. For example, three actuator assemblies 108 can be provided for a prosthetic valve having three actuators. In other embodiments, a greater or fewer number of actuator assemblies can be present.

In some embodiments, a distal end portion 116 of the shaft 106 can be sized to house the prosthetic valve in its radially compressed, delivery state during delivery of the prosthetic valve through the patient's vasculature. In this manner, the distal end portion 116 functions as a delivery sheath or capsule for the prosthetic valve during delivery,

The actuator assemblies 108 can be releasably coupled to the prosthetic valve 102. For example, in the illustrated embodiment, each actuator assembly 108 can be coupled to a respective actuator of the prosthetic valve 102. Each actuator assembly 108 can comprise a support tube, an actuator member, and a locking tool. When actuated, the actuator assembly can transmit pushing and/or pulling forces to portions of the prosthetic valve to radially expand and collapse the prosthetic valve as previously described. The actuator assemblies 108 can be at least partially disposed radially within, and extend axially through, one or more lumens of the outer shaft 106. For example, the actuator assemblies 108 can extend through a central lumen of the shaft 106 or through separate respective lumens formed in the shaft 106.

The handle 104 of the delivery apparatus 100 can include one or more control mechanisms (e.g., knobs or other actuating mechanisms) for controlling different components of the delivery apparatus 100 in order to expand and/or deploy the prosthetic valve 102. For example, in the illustrated embodiment the handle 104 comprises first, second, and third knobs 110, 112, and 114.

The first knob 110 can be a rotatable knob configured to produce axial movement of the outer shaft 106 relative to the prosthetic valve 102 in the distal and/or proximal directions in order to deploy the prosthetic valve from the delivery sheath 116 once the prosthetic valve has been advanced to a location at or adjacent the desired implantation location with the patient's body. For example, rotation of the first knob 110 in a first direction (e.g., clockwise) can retract the sheath 116 proximally relative to the prosthetic valve 102 and rotation of the first knob 110 in a second direction (e.g., counter-clockwise) can advance the sheath 116 distally. In other embodiments, the first knob 110 can be actuated by sliding or moving the knob 110 axially, such as pulling and/or pushing the knob. In other embodiments, actuation of the first knob 110 (rotation or sliding movement of the knob 110) can produce axial movement of the actuator assemblies 108 (and therefore the prosthetic valve 102) relative to the delivery sheath 116 to advance the prosthetic valve distally from the sheath 116.

The second knob 112 can be a rotatable knob configured to produce radial expansion and/or contraction of the prosthetic valve 102. For example, rotation of the second knob 112 can move the actuator member and the support tube axially relative to one another. Rotation of the second knob 112 in a first direction (e.g., clockwise) can radially expand the prosthetic valve 102 and rotation of the second knob 112 in a second direction (e.g., counter-clockwise) can radially collapse the prosthetic valve 102. In other embodiments, the second knob 112 can be actuated by sliding or moving the knob 112 axially, such as pulling and/or pushing the knob.

The third knob 114 can be a rotatable knob configured to retain the prosthetic heart valve 102 in its expanded configuration. For example, the third knob 114 can be operatively connected to a proximal end portion of the locking tool of each actuator assembly 108. Rotation of the third knob in a first direction (e.g., clockwise) can rotate each locking tool to advance the locking nuts to their distal positions to resist radial compression of the frame of the prosthetic valve, as described above. Rotation of the knob 114 in the opposite direction (e.g., counterclockwise) can rotate each locking tool in the opposite direction to decouple each locking tool from the prosthetic valve 102. In other embodiments, the third knob 114 can be actuated by sliding or moving the third knob 114 axially, such as pulling and/or pushing the knob.

Although not shown, the handle 104 can include a fourth rotatable knob operative connected to a proximal end portion of each actuator member. The fourth knob can be configured to rotate each actuator member, upon rotation of the knob, to unscrew each actuator member from the proximal portion of a respective actuator. As described above, once the locking tools and the actuator members are uncoupled from the prosthetic valve 102, they can be removed from the patient.

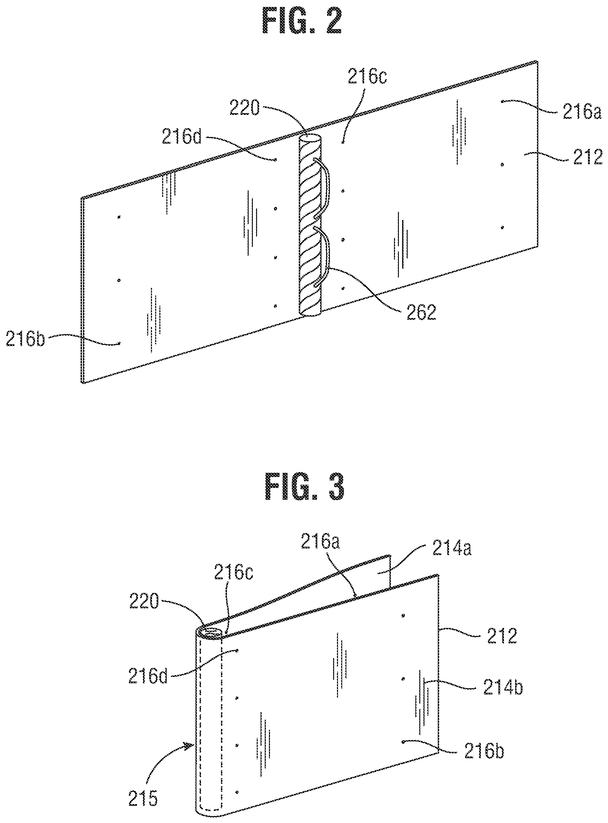

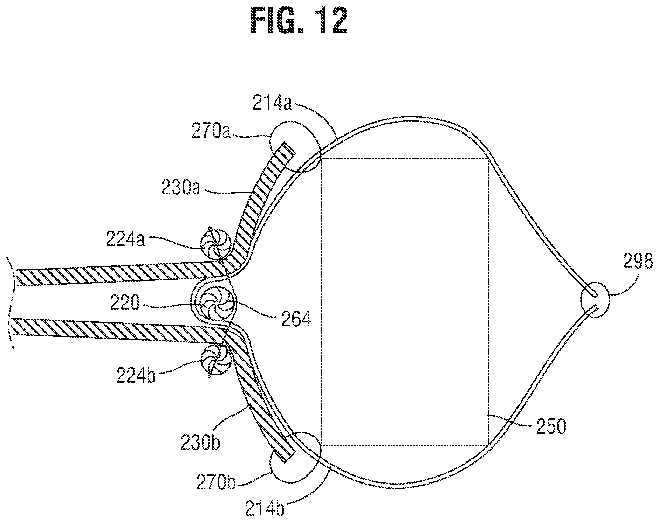

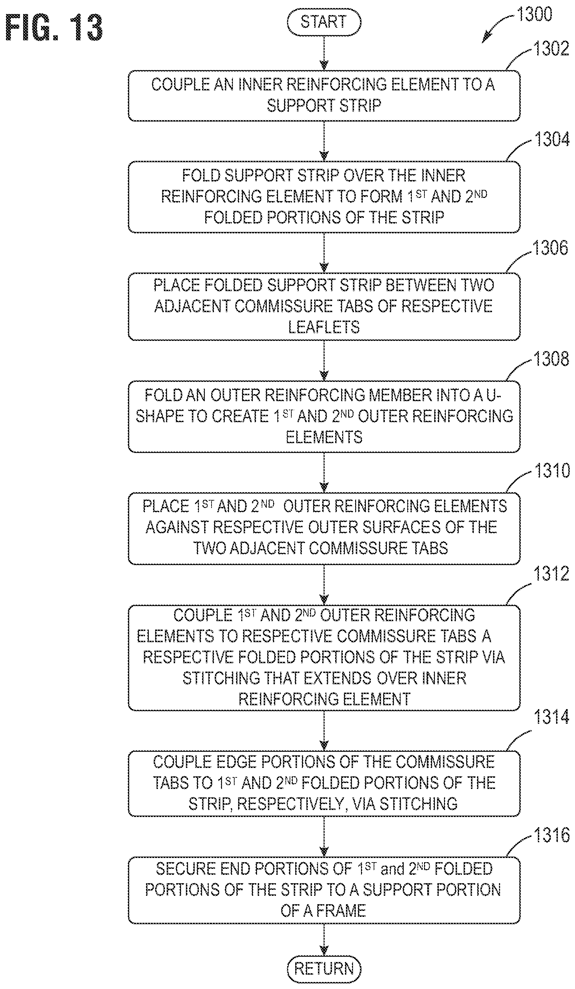

FIGS. 2-11 and 14 show example stages of assembling and mounting of a commissure to a commissure support portion (e.g., a support post) of a frame of a prosthetic valve (e.g., a prosthetic heart valve, such as prosthetic heart valve 10 of FIG. 1 and/or the prosthetic heart valve of FIGS. 12 and 13, described in more detail below). Referring to FIG. 2, an example first stage of assembly may include attaching an inner reinforcing element 220 to a support strip 212 (e.g., a fabric strip) along a vertical centerline of the strip 212. The inner reinforcing element may be attached or otherwise coupled to the support strip in any suitable manner (e.g., adhering, melting/sintering, stitching, etc.). In the illustrated example, the inner reinforcing element 220 is attached to the support strip 212 using a suture that forms stitches 262 passing through the strip 212 and the inner reinforcing element 220.

The support strip 212 may be a strip of any suitable material (e.g., fabric), which may include material that is stronger (e.g., more resilient to tearing and/or deforming) than a material used for forming the leaflets and/or the commissure tab portions of the leaflets of the prosthetic valve (not shown in FIG. 2 and described in more detail below). In one example, the support strip 212 is a polyethylene terephthalate (PET) fabric, although various other suitable biocompatible fabrics can be used.

The support strip 212 may be continuous (e.g., with no gaps and/or a substantially uniform distribution of the material forming the support strip) and may be longer in one dimension than another (e.g., having a width/length that is greater than a height of the strip). The support strip 212 may be relatively thin (e.g., having a thickness that is substantially smaller than the width and height of the strip and substantially smaller than a width or diameter of the inner reinforcing element 220).

The support strip may include one or more stitching lines or other markings, examples of which are shown at 216a, 216b, 216c, and 216d. For example, stitching lines 216a and 216b may be positioned near end portions of the support strip (e.g., adjacent vertical edges of the strip), while stitching lines 216c and 216b may be positioned near the fold region (e.g., adjacent the inner reinforcing element 220) of the support strip. The stitching lines or markings may be ink markings or other types of markings that visually indicate the locations for placing sutures during assembly. In the illustrated example, each stitching line comprises a series of spaced apart circular markings or "dots".

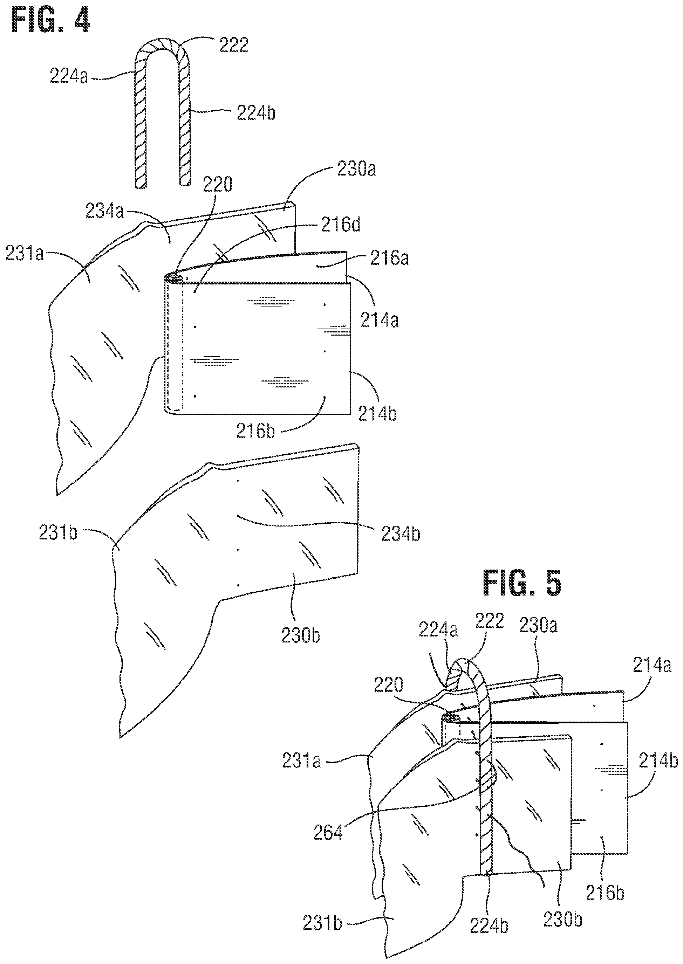

The stitching lines or markings may optionally be formed with a plurality of pre-formed apertures or openings for receiving sutures (e.g., apertures or openings that extend at least partially through the support strip). It is to be understood that the number of stitching lines illustrated herein on the support strip is exemplary, and any suitable number of stitching lines or other markings may be included on the support strip or other elements of the prosthetic valve (e.g., the commissure tabs, as described in more detail below with respect to FIG. 4) without departing from the scope of the disclosure.

The inner reinforcing element 220 can be a string, or cord, or a relatively thick suture, such as an Ethibond suture, which may be substantially wider/thicker and/or have a substantially larger diameter (e.g., at least twice as wide/thick and/or have twice the diameter) than stitching sutures 262. As shown, the inner reinforcing element 220 may be substantially the same height (or slightly shorter, such as 1-5% shorter to provide for machining tolerances and avoid extension of the inner reinforcing element past edges of the strip) as the height of the support strip 212. In other examples, the inner reinforcing element can be a relatively narrow strip of fabric, which can be folded lengthwise one or more times to increase its overall thickness. In still other examples, the inner reinforcing element can be a metal wire or a bar, such as a rectangular or cylindrical bar, formed from a metal and/or a polymer.

Referring to FIG. 3, an example second stage of assembly of the commissure may include folding the support strip 212 (e.g., widthwise) over the inner reinforcing element 220 as illustrated, to form a first folded portion 214a and a second folded portion 214b of the support strip 212. The first folded portion 214a may extend from a fold region 215 of the support strip 212 to a first terminal end of the support strip, and the second folded portion 214b may extend from the fold region 215 of the support strip to a second terminal end of the support strip. The support strip 212 may be folded symmetrically, such that opposing ends (e.g., the terminal ends) of the support strip 212 are aligned and/or corresponding stitching lines on the first and second folded portions are aligned (e.g., stitch line 216c is aligned with stitch line 216d and/or stitch line 216a is aligned with stitch line 216b).

The inner reinforcing element 220 is disposed within the fold region 215 of the support strip 212, such that the inner reinforcing element is adjacent to and/or in contact with an inner surface of the support strip. For example, the inner surface of the support strip in the fold region may be coplanar with at least a portion of the inner reinforcing element (e.g., following a curvature of an outer surface of the inner reinforcing element) and may at least partially cover (e.g., envelope) the inner reinforcing element.

Referring to FIG. 4, an example third stage of assembly of the commissure may include placing or otherwise positioning the folded support strip 212 between two adjacent commissure tabs 230a and 230b of two adjacent leaflets 231a and 231b, respectively (which can have the same overall shape of leaflets 22 of FIG. 1). In the illustrated example, the opposing ends of the first folded portion 214a and the second folded portion 214b extend beyond the respective edges of the commissure tabs 230a and 230b.

The commissure tabs 230a and 230b may include one or more stitching lines or other markings, examples of which are shown at 234a and 234b. The stitching lines 234a and 234b may be similar to the stitching lines 216a-c of the support strip. For example, the stitching lines 234a and 234b may be ink markings or other types of markings that visually indicate the locations for placing sutures during assembly and that may optionally be formed with a plurality of pre-formed apertures or openings for receiving sutures (e.g., apertures or openings that extend at least partially through the commissure tabs). The stitching lines 234a and 234b may be aligned with one another (and with the stitching lines 216c and 216d of the support strip 212) when the commissure tabs are positioned for attachment to the support strip 212.

FIG. 4 also shows an outer reinforcing member 222, which includes a first outer reinforcing element 224a and a second outer reinforcing element 224b. In the illustrated example, the outer reinforcing member 222 is a single member that is folded into a U-shape configuration to form the first and second outer reinforcing elements (e.g., the first and second outer reinforcing elements are individual elements that are coupled to one another and/or form different sections of a single continuous element). In other examples, the outer reinforcing member 222 may be discontinuous and include the first and second discrete or separate outer reinforcing elements in a spatially separated configuration (e.g., where the first and second outer reinforcing elements are spatially separated from one another).

In any of the above examples, the outer reinforcing member 222 and/or the first and second outer reinforcing elements 224a and 224b may comprise any of the materials or structures described above for the inner reinforcing member. In particular embodiments, the outer reinforcing member comprises a string, a cord, or a relatively thick suture, such as an Ethibond suture, which is substantially wider than stitching sutures 262. In some examples, the outer reinforcing member 222 may be formed of the same material and/or have a same thickness/diameter as the inner reinforcing element 220. In other examples, the outer reinforcing member 222 or portions thereof may have a different composition and/or thickness/diameter than the inner reinforcing element 220.

Referring to FIG. 5, an example fourth stage of assembly of the commissure may include placing the first and second outer reinforcing elements 224a and 224b against the outer surfaces of the commissure tabs 230a and 230b, respectively. In some examples, the first and second outer reinforcing elements may be aligned with the stitching lines 234a and 234b and initially positioned adjacent the inner reinforcing element 220 (e.g., in front of and closer to end portions of the commissure tabs/support strip than the inner reinforcing element 220). In other examples, the first and second outer reinforcing elements may be aligned with the inner reinforcing element 220. A suture may be used to form stitches 264 that extend through, in order: the first outer reinforcing element 224a, the commissure tab 230a (e.g., through the stitching line 234a), the first folded portion 214a (e.g., through the stitching line 216c), the second folded portion 214b (e.g., through the stitching line 216d), the commissure tab 230b (e.g., through the stitching line 234b), and the second outer reinforcing element 224b in order to attach the first and second outer reinforcing elements to the commissure tabs and support strip. As shown, the stitches 264 can be in-and-out stitches. although other stitching techniques can be used. The commissure tabs 230a and 230b and the support strip 212 may together form the commissure, which can then be mounted to a frame of a prosthetic valve, as further described below.

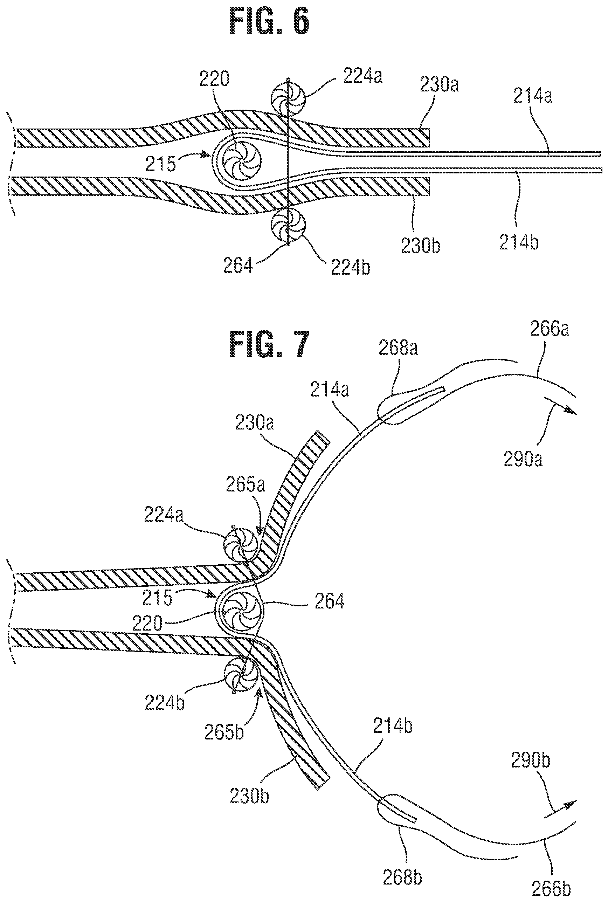

FIG. 6 is a cross-sectional view of the commissure after attaching the first and second outer reinforcing elements 224a and 224b to the commissure tabs 230a and 230b and the folded support strip 212. As shown, the inner reinforcing element 220 is secured between the fold region 215 of the support strip 212 and the stitches 264 (one of which is shown in FIG. 6). The stitches 264 extend behind the inner reinforcing element 220, such that the stitches 264 are adjacent an opposite side of the inner reinforcing element 220 than the fold region 215 of the support strip 212. Accordingly, the inner reinforcing element 220 is positioned between inner surfaces of the commissure tabs 230a and 230b, while the first and second outer reinforcing elements 224a and 224b are positioned adjacent outer surfaces of the commissure tabs 230a and 230b, respectively (e.g., on opposite sides of the commissure tabs than the inner reinforcing element).

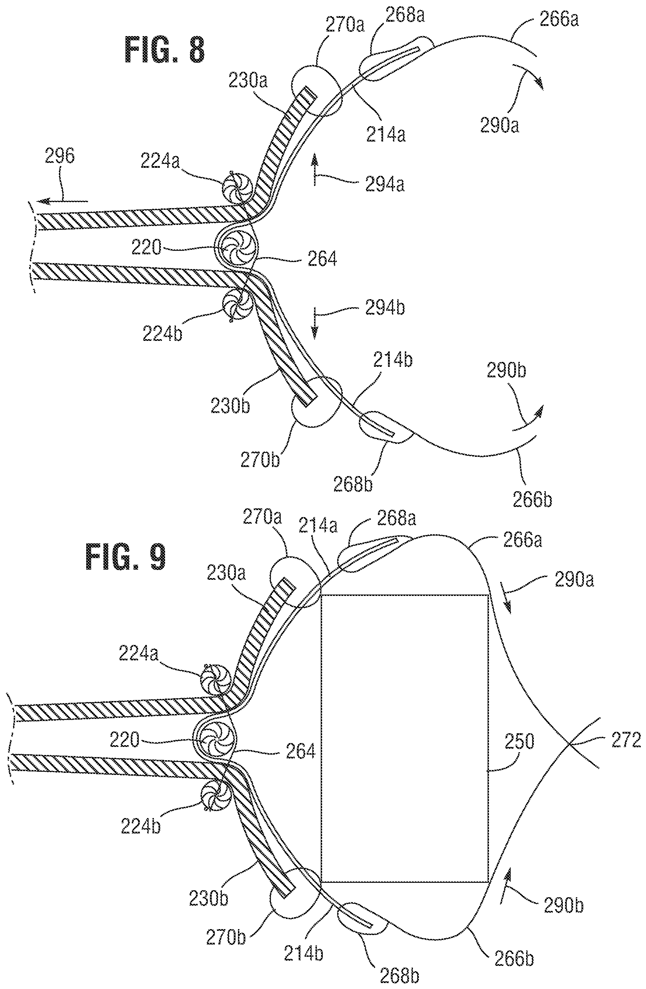

As described above, the commissure may be attached and/or mounted to a commissure support portion (e.g., a support strut/post) of a frame of the prosthetic valve. FIG. 7 shows one example of a fifth stage of assembling the commissure, including attaching sutures 266a and 266b to the first folded portion 214a of the support strip and the second folded portion 214b of the support strip, respectively. The sutures 266a and 266b are stitched to the first folded portion 214a and the second folded portion 214b via stitches 268a and 268b, respectively, along sections of the first folded portion and the second folded portion that extend beyond the edges of commissure tabs 230a and 230b (e.g., through stitch lines 216a and 216b, respectively). The opposite end portions of the sutures 266a and 266b are then pulled in directions indicated by arrows 290a and 290b, respectively (e.g., to extend around the support strut, as described below with respect to FIG. 9).

As shown, the stitch(es) 264 is anchored to the inner reinforcing element 220 and holds both outer reinforcing elements 224a and 224b that are holding the commissure tabs 230a and 230b. As the commissure tabs of the leaflets pull on the outer reinforcing elements, the outer reinforcing elements are pulled closer to each other, creating a vice action on the commissure tabs of the leaflets. This happens because the stitch(es) 264 is anchored at a central hinge point on inner reinforcing element 220. Furthermore, the pulling of the sutures 266a and 266b (e.g., to extend around the support strut) may cause end portions of the commissure tabs 230a and 230b to fold back (e.g., in opposing directions to one another) and partially over the first and second outer reinforcing elements 224a and 224b, respectively, thereby bringing the first and second outer reinforcing elements toward and optionally into alignment with the inner reinforcing element 220. Accordingly, the first and second outer reinforcing elements may be positioned in respective fold regions 265a and 265b of the commissure tabs 230a and 230b. The folding of the commissure tabs may further cause the stitch 264 to come into contact with the inner reinforcing element 220, deforming the stitch to form a slight fold in the stitch opposite the fold region 215 of the support strip 212. Accordingly, the inner reinforcing element 220 may be secured between the fold region 215 of the support strip 212 and a fold of the stitch 264. It is to be understood that in other examples, the outer reinforcing elements 224a and 224b and/or the stitch 264 may be positioned such that the stitch 264 extends behind the inner reinforcing element 220 (e.g., between the inner reinforcing element 220 and the fold region 215 of the folded support strip 214) or through the inner reinforcing element 220.

Referring briefly to FIG. 8, an optional additional stage of assembling the commissure may include attaching edge portions of commissure tabs 230a and 230b to the first folded portion 214a and the second folded portion 214b, respectively, for example via supplemental stitches 270a and 270b. The supplemental stitches 270a and 270b may be attached at a location of the first folded portion between stitching lines 216a and 216c and at a location of the second folded portion between stitching lines 216b and 216d in order to supplement the attachment of the support strip to the commissure tabs that is provided by the stitch 264 near the fold region of the support strip.

Referring to FIG. 9, one example of a sixth stage of assembling the commissure includes securing the commissure to a support strut 250 of a frame of the prosthetic valve. As shown, the sutures 266a and 266b are pulled around the support strut 250 in the opposing directions indicated by arrows 290a and 290b, respectively, to reach one another. The sutures 266a and 266b may be secured to one another around the support strut 250 by stitching or tying the sutures together at tie 272, which may be positioned on an opposite side of the support strut from the fold region of the support strip 212 (e.g., in alignment with the fold region).

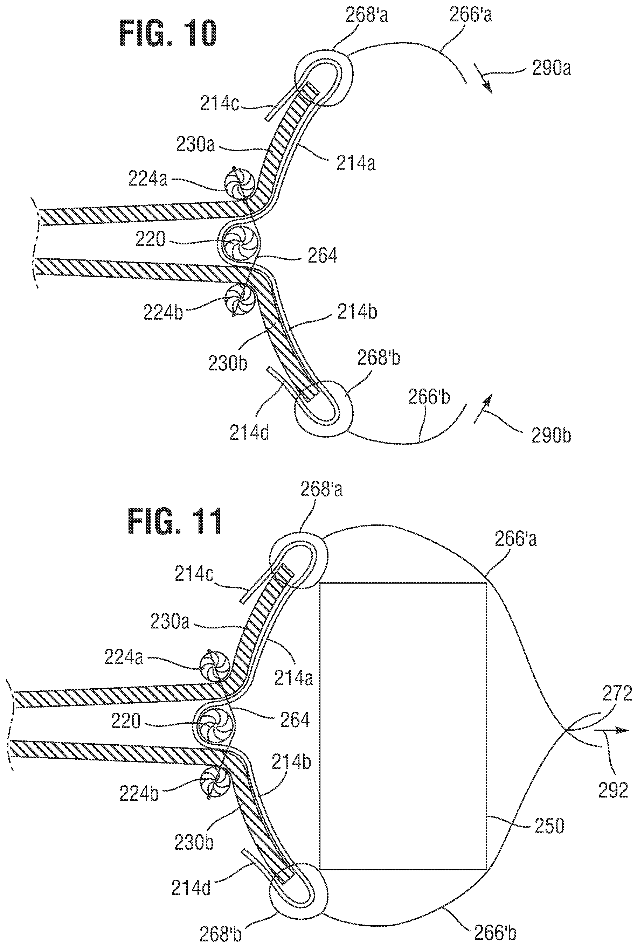

Referring to FIG. 10, another example of the fifth stage of assembling the commissure may include folding opposing end portions of the support strip 212 over corresponding end portions of the adjacent commissure tabs 230a and 230b. For example, each of the first folded portion 214a and the second folded portion 214b may be folded around outer edge regions of the commissure tabs 230a and 230b, respectively, to form a first flap 214c and a second flap 214d, respectively. A suture 266'a may be stitched through the first folded portion 214a and the commissure tab 230a through stitch 268'a, and a suture 266'b may be stitched through the second folded portion 214b and the commissure tab 230b through stitch 268'b, such that stitches 268'a and 268'b fulfill the roles of both stitches 268a, 268b and 270a, 270b as described above with respect to FIGS. 7-9. For example, suture 266'a may be stitched through the first flap 214c, the commissure tab 230a, and the first folded portion 214a through stitch 268' and suture 266'b may be stitched through the second flap 216d, the commissure tab 230b, and the second folded portion 214b through stitch 268'b.

Referring to FIG. 11, another example of the sixth stage of assembling the commissure is similar to the sixth stage described above with respect to FIG. 9, and includes pulling the sutures 266'a and 266'b around the support strut 250 and attaching the sutures 266'a and 266'b to one another at tie 272. In some embodiments, the first folded portion 214a and the second folded portion 214b are both stitched to a single suture instead of two separate sutures, such as sutures 266a, 266b or 266'a, 266'b, wherein both ends of the single suture are secured to one another by stitching or tying them together at tie 272.

Referring to FIG. 12, another example of the fifth and sixth stages of assembling the commissure may be performed for embodiments in which the first folded portion 214a of the support strip 212 and the second folded portion 214b of the support strip 212 are long enough such that opposing end portions of the first and second folded portions may be pulled around the support strut 250 and directly attached to each other (e.g., via stitching or tying the end portions together). For example, respective end portions of the first folded portion 214a and the second folded portion 214b may be pulled in opposite directions indicated by arrows 290a and 290b around the support strut 250 and tied directly to one another and/or coupled via suture 298 at a region opposite the fold region of the support strip 212. The first and second folded portions may optionally be further attached to the commissure tabs via supplemental sutures 270a and 270b, respectively, as described above with respect to FIG. 8.

Advantageously, the resulting commissure configurations described herein effectively move the direction of force carrying axes away from the commissure tabs 230a and 230b. For example, when the first folded portion 214a and the second folded portion 214b of the support strip 212 are pulled around the support strut 250 in directions indicated by arrows 290a and 290b, either directly or via sutures 266a and 266b or 266'a and 266'b, or when suture or tie 272 is pulled in direction 292 (see FIG. 11), the pull forces are translated via stitches 268a and 268b or 268'a and 268'b to the edges of the support strip 212, which may be formed of a high-durability material (e.g., a cloth, such as a PET fabric) or other material, instead of acting on the suturing regions of the commissure tabs 230a and 230b, thereby reducing stress concentrations on the commissure tabs and associated leaflets.

Moreover, pulling the first folded portion 214a and the second folded portion 214b in directions indicated by arrows 290a and 290b, or pulling the tie 272 in direction 292, may translate at least partially to pull forces acting on the stitch 264 and the fold region 215 of the support strip 214 in a combination of circumferential directions (e.g., direction 294a adjacent its extension through the first outer reinforcing element 224a, direction 294b adjacent its extension through the second outer reinforcing element 224b) and radial directions (e.g., direction 292) . These pull forces may act to tighten and vertically straighten the stitch 264, which may result in a general displacement of the inner reinforcing element 220 in direction 296 (see FIG. 8, e.g., away from the support post 250), as well as to tighten and vertically straighten the fold region 215 of the support strip 214, which may result in a general displacement of the inner reinforcing element 220 in a direction opposite of direction 296 (e.g., toward the support post 250).

The pull forces described above may also result in general displacement of the outer reinforcing elements 224a and 224b (e.g., in the direction 296 for lateral pull forces on the support strip 214). The net effect of the pull forces may cause a general displacement of the inner and/or outer reinforcing elements that is based on parameters of the pull forces (e.g., source/location, magnitude, direction, etc.), which may in turn be based on parameters of the configuration (e.g., support post size/shape, nature of attachment of the commissure to the frame, commissure tab length, support strip length, etc.). In any scenario, however, as the pull forces act on the stitch 264 and support strip 214 to translate displacement to the inner reinforcing element 220 and/or outer reinforcing elements 224a and 224b, the configuration may further reduce stresses acting directly on commissure tabs 230a and 230b. Similarly, pull forces on the leaflets in the direction 296 (e.g., derived by diastolic pressure while the prosthetic valve is deployed in a patient), may be translated to the stitch 264 and support strip 214 to effect displacement of the inner and/or outer reinforcing elements 220, 224a, and 224b, thereby reducing stresses acting directly on the commissure tabs 230a and 230b.

In particular embodiments, a prosthetic valve can be assembled by first forming a leaflet assembly and then assembling the leaflet assembly onto the frame (e.g., frame 12). The leaflet assembly can be formed by connecting all pairs of adjacent commissure tabs of all of the leaflets of the prosthetic valve by implementing at least the steps described above in connection with FIGS. 2-6. Typically, the prosthetic valve has three leaflets (although a greater or fewer number of leaflets may be used), in which case the leaflet assembly has three pairs commissure tabs secured to each other in the manner shown in FIG. 6.

An inner skirt (e.g., inner skirt 20) can be placed inside of the frame and secured thereto with sutures (e.g., suture loops) 44, as shown in FIG. 1 and described above. The leaflet assembly can then be placed inside of the frame. Each commissure can be secured to a respective commissure support portion (e.g., a support strut 250 or an actuator 80) using one or more of the techniques described above in connection with FIGS. 7-12. The cusp edge portion of each leaflet can be secured to the inner skirt 20 by stitching the cusp edge portion of each leaflet to the inner skirt to form stitching 42.

It should be understood that some of the steps shown in FIGS. 7-12 optionally can be performed prior to placing the leaflet assembly inside of the frame. For example, the sutures 266a, 266b can be threaded through the support strip 212 as shown in FIG. 7 prior to placing the leaflet assembly inside of the frame. Similarly, sutures forming stitches 270a, 270b (FIGS. 8-9 and 12) or sutures forming stitches 266'a, 266'b, 268'a, 268'b (FIGS. 10-11) can be added prior to placing the leaflet assembly inside the frame.

FIG. 13 is a flow chart of an example method 1300 of assembling a commissure of a prosthetic valve and mounting the commissure on a corresponding support portion of a frame of a prosthetic valve. For example, at least a portion of method 1300 may be performed to assemble the commissure configurations as illustrated in FIGS. 1-12. It is to be understood that one or more of the elements of method 1300 may be omitted or performed in a different order without departing from the scope of the disclosure. Method 1300 may be iteratively performed to form a plurality of commissures with a plurality of leaflets of a prosthetic valve (e.g., a prosthetic heart valve). Each commissure may be formed by pairing a first commissure tab of a first leaflet with an adjacent, second commissure tab of a second leaflet, then performing at least a portion of method 1300 for the respective pair of commissure tabs. The prosthetic valve may be assembled by mounting each of the resulting commissures to respective support portions (e.g., support struts) of a frame of the prosthetic valve.

As illustrated in FIG. 13, at 1302, the method includes coupling an inner reinforcing element to a support strip. An example of coupling the inner reinforcing element to the support strip is shown and described above with respect to FIG. 2. At 1304, the method includes folding the support strip over the inner reinforcing element to form first and second folded portions of the support strip. An example of folding the support strip over the inner reinforcing element is shown and described above with respect to FIG. 3.

At 1306, the method includes placing the folded support strip between two adjacent commissure tabs of respective leaflets. As shown in FIG. 4, the folded support strip may be positioned in alignment with a cusp region of the commissure tabs, and stitching lines may be used to assist in the alignment (e.g., stitching lines near the cusp region of the commissure tabs may be aligned with stitching lines near the fold region of the support strip).

In order to provide a robust attachment of the support strip to the commissure tabs, an outer reinforcing member may be used to reinforce the location of attachment. In examples where the outer reinforcing member is a single reinforcing member (e.g., a single, thick suture), the member may be prepared for use in the commissure by folding the outer reinforcing member into a U-shape to create first and second outer reinforcing elements, as indicated at 1308. In other examples, the first and second outer reinforcing elements may be formed in another manner and/or may be separate or discrete elements (e.g., 1308 may be omitted or replaced with an alternative outer reinforcing member preparation stage).

At 1310, the method includes placing the first and second outer reinforcing elements against respective outer surfaces of the two adjacent commissure tabs. At 1312, the method includes coupling the first and second outer reinforcing elements to respective commissure tabs and respective folded portions of the strip via stitching that extends over the inner reinforcing element. FIG. 5 shows an example of the above-described positioning and coupling of the outer reinforcing elements. The cross-sectional view of FIG. 6 illustrates an example of the extension of the stitching behind (radially outward of) the inner reinforcing element. As illustrated, in examples in which the first and second outer reinforcing elements are part of a single, U-shaped outer reinforcing member, a fold region of the outer reinforcing member may extend across edge portions of the two adjacent commissure tabs and the folded support strip. Accordingly, the fold region of the outer reinforcing member may be at least partially aligned with the stitching that extends across the inner reinforcing element.

At 1314, the method includes optionally coupling edge portions of the commissure tabs to first and second folded portions of the support strip, respectively, via additional stitching. As described above with respect to FIG. 8, which shows an example of the coupling of the edge portions of the commissure tabs to the folded portions of the support strip, the additional point of coupling between these elements may provide supplemental stress/force distribution to reduce stress/force on the commissure tabs and/or associated leaflets. For example, when subjected to pulling forces, the support strip may generally be vulnerable to tearing or fraying. Folding the support strip around the commissure tabs may create a robust attachment, as the commissure tab material positioned between the fold of the support strip by the suture loop may prevent the breaking away of material (e.g., threads) forming the support strip. The coupling may also help to keep the edge portions of the commissure tabs from moving independently from the folded support strip, which may further reduce wear or other complications arising from such movement.

At 1316, the method includes securing the end portions of the first and second folded portions of the support strip to a support portion (e.g., support strut 250) of a frame of the prosthetic valve, which may include coupling the end portions of the first and second folded portions of the support strip to one another around the support portion of the frame. Examples of this coupling are shown in FIGS. 9, 11, and 12, and may include direct coupling (e.g., where end portions of the first and second folded portions of the support strip are directly sutured, adhered, or otherwise coupled and/or in contact with one another), indirect coupling via a single suture, indirect coupling via multiple sutures (e.g., a first suture attached to the first folded portion and a second suture attached to the second folded portion), and/or any other suitable coupling mechanism. In some examples, direct attachment of the end portions of the support strip to one another may be reinforced by a further suture or other coupling mechanism applied to a region where the end portions of the support strip are coupled to one another (e.g., stitching a suture through the end portions of the support strip in the region of direct attachment).

Securing the commissure to the support portion of the frame may utilize any suitable coupling mechanisms, such as stitching (e.g., shoelace stitching), knotting/tying (e.g., square knotting), adhering, sintering, and/or any other mechanism or combination of mechanisms. A non-limiting, illustrative example of a coupling method may include making tight shoelace stitches with a suture starting at an outflow edge of the support portion of the frame, making a square knot, using remaining suture tails to make a shoelace stitch under the resulting junction (e.g., the first and last stitch sutures may be on the outer side of the tissue-skirt subassembly), making two square knots, and cutting suture tails to a selected length.

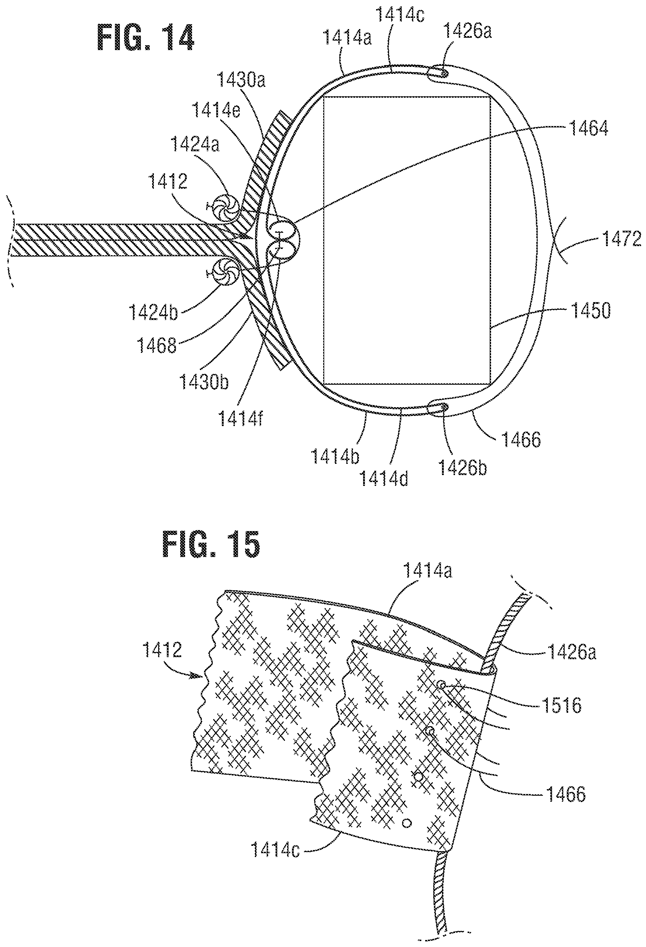

FIG. 14 is another example of assembling a commissure, where the commissure of the example of FIG. 14 has a reinforced zone formed by folded end portions of a support strip and edge reinforcing members. The stitched end portions of the support strip reinforce the commissure and reduce the risk of cloth tear, without using an attached external component, such as an inner reinforcing member (e.g., formed of a suture or other material). The folded support strip of the assembly shown in FIG. 14 also forms a double-layered structure, thereby doubling the total thickness of the material disposed between leaflets associated with the commissure.

The configuration of FIG. 14 is similar to that of FIGS. 9 and 11, however, the configuration of FIG. 14 does not include an inner reinforcing element attached to a vertical centerline of a support strip. In the configuration of FIG. 14, a first portion 1414a of the support strip 1412 extends (e.g., from the vertical centerline of the support strip 1412) in one direction, and is folded (e.g., widthwise) over an edge reinforcing element 1426a (shown and described in more detail with respect to FIG. 15) to form a third portion 1414c extending back toward the vertical centerline of the support strip 1412. A second portion 1414b of the support strip extends (e.g., from the vertical centerline of the support strip) in an opposing direction to that of portion 1414a, and is folded (e.g., widthwise) over an edge reinforcing element 1426b to form a fourth portion 1414d extending back toward the vertical centerline of the support strip 1412. The edge reinforcing elements 1426a and 1426b can be sutures or pieces of fabric, and may be thinner than the Ethibond or other material used as an inner reinforcing element 220 in FIG. 2 and/or thinner than the outer reinforcing elements 1424a and 1424b. However, in other implementations, elements 1426a, 1426b can be Ethibond sutures.

The support strip end portions 1414e and 1414f, at the ends of portions 1414c and 1414d, respectively, are stitched together via stitches 1468. According to some embodiments, the remaining sections of support strip end portions 1414e and 1414f extending from the stitches 1468, are further folded over stitches 1468. In this example, a reinforced region is formed along the vertical centerline of the support strip 1412, by stitching end portions of the support strip 1412 itself instead of using an additional external component, such as an inner reinforcing element 220 of FIG. 2.

The resulting folded support strip 1412 is then positioned between the commissure tabs 1430a and 1430b, having stitched end portions 1414e and 1414f placed therebetween. A suture is then used to form stitches 1464 through (e.g., in order) the first outer reinforcing element 1424a, commissure tab 1430a, folded portions 1414a and 1414c, folded portions 1414d and 1414b, commissure tab 1430b and the second outer reinforcing element 1424b. In this way, the stitches 1464 extend over the reinforced region of the support strip, such that the reinforced region of the support strip is positioned between the stitches 1464 and the commissure tabs 1430a and 1430b (e.g., the inner surfaces of the commissure tabs, in a region where the commissure tabs meet one another). In other examples, the stitches 1464 may pass through the extended tabs in the reinforced region.

Suture 1466 is then stitched through folded portions 1414a and 1414c and through folded portion 1414d and 1414b on opposite sides of a support post 1450, finally securing the commissure to the support post 1450 via ties 1472. It is to be understood that the commissure may be secured to the support post 1450 using any suitable mechanism, including the example approaches described herein (e.g., in FIGS. 9, 11, and 12). In the illustrated example, the suture 1466 passes through the folded portions 1414a-d behind (e.g., toward the commissure tabs) the respective edge reinforcing elements 1426a and 1426b. In other examples, the suture 1466 and/or an additional suture attached thereto may pass through the edge reinforcing elements 1426a and 1426b and/or in front of (e.g., toward the tie 1472 and away from the commissure tabs) the edge reinforcing elements 1426a and 1426b.

FIG. 15 is a detailed view of a portion of the support strip 1412 of FIG. 14 (e.g., portions 1414a and 1414c) folded around the edge reinforcing element 1426a. According to some embodiments, the folded portions 1414a and 1414c comprise stitching lines 1516, adjacent the edge reinforcing element 1426a, and the folded portion 1414b and 1414d (not shown in FIG. 15) comprise stitching lines adjacent the edge reinforcing element 1426b. The stitching lines 1516 for folded portions 1414a and 1414c may be aligned over each other when the support strip 1412 is folded over the edge reinforcing element 1426a, and the stitching lines for folded portions 1414b and 1414d may be aligned over each other when the support strip 1412 is folded over the edge reinforcing element 1426b.

According to some embodiments, support strip end portions 1414e and 1414f comprise stitching lines adjacent to their respective edges. The stitching lines of the support strip end portions 1414e and 1414f may be aligned over each other when stitched together along the vertical centerline of the support strip 1412.

In some examples, at least one of stitching lines along portions 1414a, 1414b, 1414c, 1414d, 1414e, and 1414f comprises an external marking, such as an ink marking, visually indicating suturing locations for a user assembling the commissure. In additional or alternative examples, at least one of the stitching lines along portions 1414a, 1414b, 1414c, 1414d, 1414e, and 1414f may be formed with a plurality of apertures or openings, through which a needle carrying sutures can pass.

In the illustrated example of FIG. 15, the edge reinforcing element 1426 is longer than the height of the support strip 1412, and thus extends past edges of the support strip in a vertical direction (e.g., along a longitudinal axis of the edge reinforcing element). In such examples, the edge reinforcing element 1426a may be reinforced by tying or otherwise coupling ends of the edge reinforcing element 1426a to corresponding ends of edge reinforcing element 1426b (e.g., a top end of edge reinforcing element 1426a may be coupled to a top end of edge reinforcing element 1426b, and a bottom end of edge reinforcing element 1426a may be coupled to a bottom end of edge reinforcing element 1426b). In other examples, the edge reinforcing element 1426a may be reinforced by tying or otherwise coupling opposing ends of the edge reinforcing element 1426a to one another (e.g., a top end of edge reinforcing element 1426a may be coupled to a bottom end of edge reinforcing element 1426a, such that the edge reinforcing element 1426a loops around an outer surface of the support strip 1412 in an edge fold region of the support strip 1412).

In additional or alternative examples to those described above, sutures may be run through the edge reinforcing elements to lock the elements in place. For example, sutures 1466/1472 and/or sutures that are supplemental to sutures 1466/1472 may be configured to pass through the edge reinforcing elements 1426a and 1426b. Such suturing may be independent of or used in combination with either example of edge reinforcing element end coupling described above and/or may be used in examples where the edge reinforcing element is sized so as to not extend beyond edges of the support strip 1412.

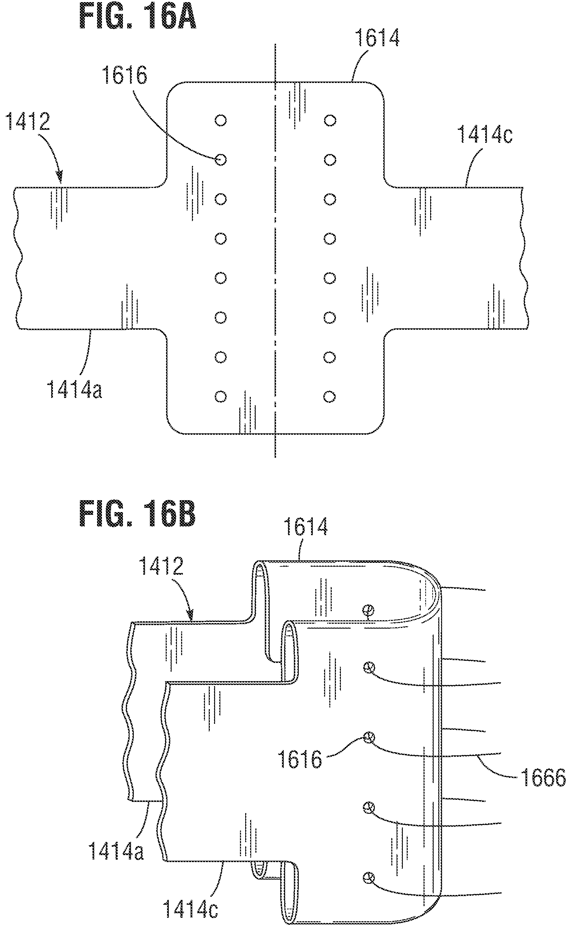

In further additional or alternative examples, instead of using an edge reinforcing element 1426a and/or 1426b, the support strip 1412 may be configured to be wider (e.g., on both sides in the axial direction of the associated valve) and folded so as to create a double layer with no free edge of the cloth. For example, FIGS. 16A and 16B show an example configuration of support strip 1412 that includes an edge reinforcing region 1614 positioned between portions 1414a and 1414c (e.g., positioned in an edge fold region of the support strip. Although only one edge reinforcing region 1614 is shown in the views of FIGS. 16A and 16B, it is to be understood that the support strip 1412 may include another, similarly-configured/dimensioned edge reinforcing region positioned between support strip portions 1414b and 1414d (which are shown in FIG. 14).

FIG. 16A shows a first view of an unfolded edge reinforcing region 1614. As shown, a height of the support strip 1412 (e.g., in the axial direction of the valve in which the support strip is included) is increased in the edge reinforcing region 1614 on both upper and lower regions of the support strip. FIG. 16B shows a second view of the edge reinforcing region 1614 folded over itself to reinforce an edge fold region of the support strip 1412. The edge reinforcing region 1614 may be used in some examples in place of the edge reinforcing elements 1426a/b, or in other examples in combination with/in addition to the edge reinforcing elements 1426a/b.

The edge reinforcing region 1614 also optionally includes a plurality of stitching lines 1616, which may be similar in configuration to stitching lines 1516 of FIG. 15. For example, the stitching lines 1616 may comprise an external marking, such as an ink marking, visually indicating suturing locations for a user assembling the commissure and/or may be formed with a plurality of apertures or openings, through which a needle carrying sutures (e.g., sutures 1666 in FIG. 16B) can pass.

The stitching lines 1616 may be used for alignment purposes when folding the edge reinforcing region 1614 over itself. For example, for each end portion of the edge reinforcing region 1614 (e.g., where the end portions extend from a center of the edge reinforcing region outward in opposing directions along a longitudinal axis of the edge reinforcing region, past respective edges of the support strip portions 1414a and 1414c), one or more markings and/or apertures/openings in an outer row(s) of the respective end portion of the edge reinforcing region may be aligned with one or more corresponding markings and/or apertures/openings in an inner (e.g., nearer to a center of the edge reinforcing region) row(s) of the respective end portion of the edge reinforcing region. In this way, the end portions of the edge reinforcing region of the support strip may be folded toward one another and each end portion may at least partially overlap itself when in a folded state (e.g., as shown in FIG. 16B). It is to be understood that other folding arrangements may be used to create the folded edge reinforcing region 1614 without departing from the scope of the disclosure.

The disclosed configurations described with respect to FIGS. 14, 15, 16A, and 16B include a reinforced region along the vertical centerline of the support strip 1412, by stitching end portion of the support strip 1412 itself instead of using an additional external component, such as inner reinforcing element 220 of FIG. 2. In the disclosed configuration, the folded support strip 1412 forms a double-layered structure, such that the total thickness of the support strip 1412 disposed between the commissure tabs 1430a and 1430b and the post 1450 is doubled, relative to the single layered support strip 212 according to FIG. 2.

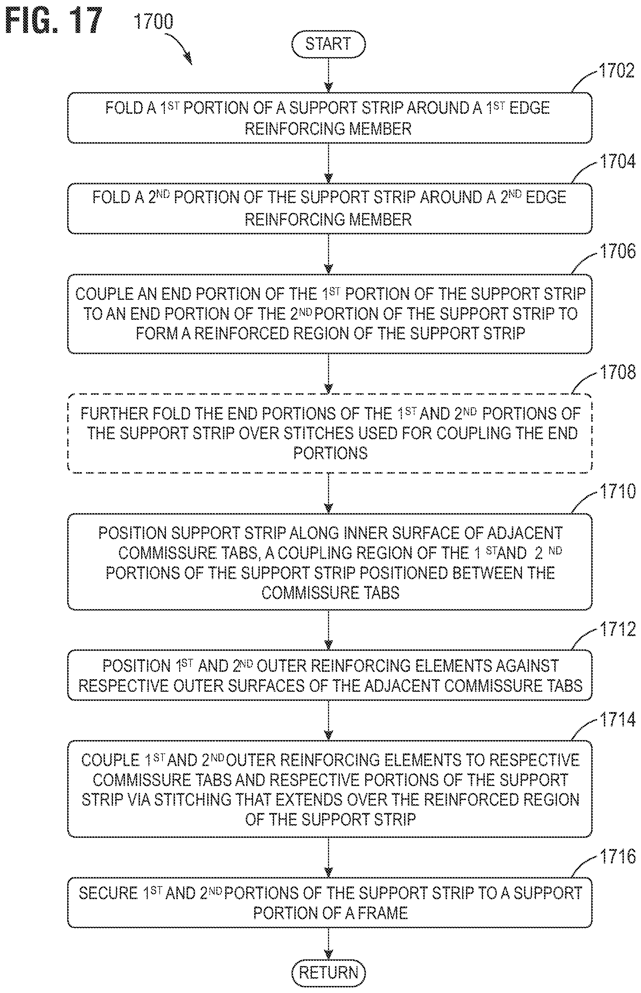

FIG. 17 is a flow chart of an example method 1700 for assembling a commissure of a prosthetic valve using edge reinforcing members and mounting the commissure on a corresponding support portion of a frame of a prosthetic valve. For example, at least a portion of method 1700 may be performed to assemble the commissure configurations as illustrated in FIGS. 14 and 15. It is to be understood that one or more elements of method 1700 may be omitted or performed in a different order without departing from the scope of the disclosure. Method 1700 may be iteratively performed to form a plurality of commissures with a plurality of leaflets of a prosthetic valve (e.g., a prosthetic heart valve). Each commissure may be formed by pairing a first commissure tab of a first leaflet with an adjacent, second commissure tab of a second leaflet, then performing at least a portion of method 1700 for the respective pair of commissure tabs. The prosthetic valve may be assembled by mounting each of the resulting commissures to respective support portions (e.g., support struts/posts) of a frame of the prosthetic valve.

As illustrated in FIG. 17, at 1702, the method includes folding (e.g., widthwise) a first portion of a support strip around a first edge reinforcing element/member. An example of folding the first portion of a support strip around a first edge reinforcing element/member is shown and described above with respect to FIG. 14 and in more detail with respect to FIG. 15.

At 1704, the method includes folding (e.g., widthwise) a second portion of the support strip around a second edge reinforcing element/member. For example, the second portion of the support strip may be folded around the second edge reinforcing element/member in a similar manner to the folding of the first portion of the support strip around the first edge reinforcing element/member as shown and described above with respect to FIG. 15. The second portion of the support strip may extend in an opposite direction from a vertical centerline of the support strip relative to the first portion of the support strip, for example as shown and described above with respect to FIG. 14. In some examples, once the commissure is assembled and/or mounted to a support structure, the second edge reinforcing element/member may be substantially in alignment with the first edge reinforcing element/member (e.g., the second edge reinforcing element may be on an opposite side of the support structure from the first edge reinforcing element).

At 1706, the method includes coupling an end portion of the first portion of the support strip to an end portion of the second portion of the support strip to form a reinforced region of the support strip. An example of coupling the end portions of the support strip to form the reinforced region is shown and described above with respect to FIG. 14. For example, stitch 1468 of FIG. 14 is one example of an element used to couple the end portions of the support strip to one another. In this way, each end portion of the support strip may extend (e.g., from a vertical centerline of the support strip) toward and then around a respective edge reinforcing element, then back toward a center of the support strip (e.g., such that the portions of the support strip are folded over itself around the edge reinforcing element) to meet the other end portion of the support strip.

At 1708, the method optionally includes further folding the end portions of the first and second portions of the support strip over stitches used for coupling the end portions. An example of the folding of the end portions over themselves and thus over stitches used for coupling the end portions to one another is shown and described above with respect to FIG. 14. For example, the end portions of the support strip 1412 are shown as being folded over (e.g., in opposite directions to one another) the stitch 1468 in FIG. 14.

At 1710, the method includes positioning the support strip along an inner surface of adjacent commissure tabs, such that a coupling region of the first and second portions of the support strip (e.g., the reinforced region of the support strip) is positioned between the commissure tabs. An example of the positioning of the support strip is shown and described above with respect to FIG. 14. For example, in FIG. 14, the first portion of the support strip is shown as being positioned at least partially in contact with and/or adjacent to an inner surface of the first commissure tab 1430a and the second portion of the support strip is shown as being positioned at least partially in contact with and/or adjacent to an inner surface of the second commissure tab 1430b. The reinforced region of the support strip, formed by the end portions of the support strip folded over stitch 1468, is shown in FIG. 14 as being approximately aligned with (e.g., positioned over) a location where the commissure tabs 1430a and 1430b meet (e.g., between the commissure tabs).

At 1712, the method includes positioning the first and second outer reinforcing elements against respective outer surfaces of the adjacent commissure tabs. At 1714, the method includes coupling the first and second outer reinforcing elements to respective commissure tabs and respective portions of the support strip via stitching that extends over the reinforced region of the support strip. An example of the positioning of the outer reinforcing elements and the coupling of these elements to the support strip is shown and described above with respect to FIG. 14. Additionally, FIGS. 5 and 6 show an example of positioning and coupling outer reinforcing elements that may also be used for this portion of method 1700. The positioning and coupling of the outer reinforcing elements may be performed in a similar manner to the placing and coupling described above at 1310 and 1312 of FIG. 13, where the commissure includes and utilizes a reinforced region (formed as described above at 1706/1708) in an analogous manner to the inner reinforcing element described with respect to FIG. 13 (and shown in FIGS. 5 and 6).

At 1716, the method includes securing first and second portions of the support strip to a support portion of a frame of the prosthetic valve (e.g., support structure 1450 of FIG. 14). An example of this securing is shown using suture 1466 in FIG. 14, however, any suitable mechanism for securing the first and second portions of the support strip may be used. For example, direct coupling (e.g., where portions of the first and second folded portions of the support strip are directly sutured, adhered, or otherwise coupled and/or in contact with one another), indirect coupling via a single suture, indirect coupling via multiple sutures (e.g., a first suture attached to the first portion of the support strip and/or first edge reinforcing element and a second suture attached to the second portion of the support strip and/or second edge reinforcing element), and/or any other suitable coupling mechanism. In some examples, direct attachment of the portions of the support strip to one another may be reinforced by a further suture or other coupling mechanism applied to a region where the portions of the support strip are coupled to one another (e.g., stitching a suture through the portions of the support strip in the region of direct attachment).

Securing the commissure to the support portion of the frame may utilize any suitable coupling mechanisms, such as stitching (e.g., shoelace stitching), knotting/tying (e.g., square knotting), adhering, sintering, and/or any other mechanism or combination of mechanisms. A non-limiting, illustrative example of a coupling method may include making tight shoelace stitches with a suture starting at an outflow edge of the support portion of the frame, making a square knot, using remaining suture tails to make a shoelace stitch under the resulting junction (e.g., the first and last stitch sutures may be on the outer side of the tissue-skirt subassembly), making two square knots, and cutting suture tails to a selected length.

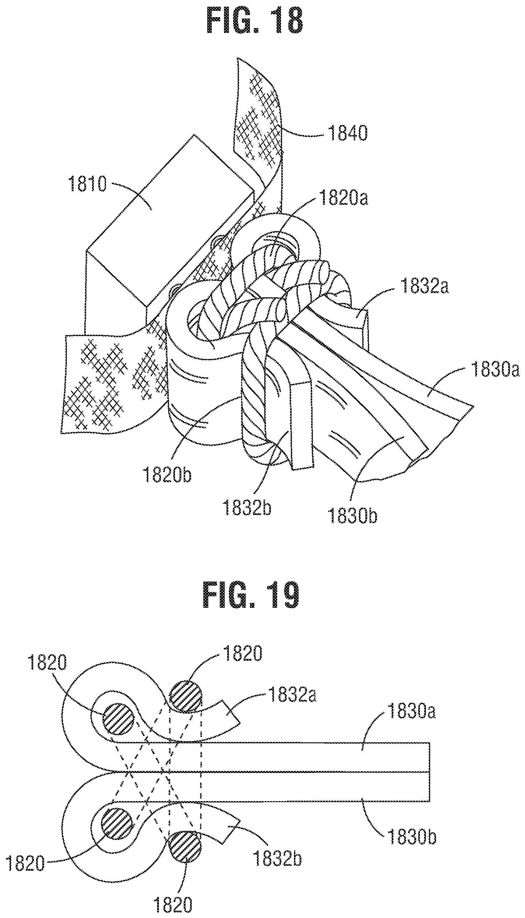

FIG. 18 is another example of assembling a commissure of a prosthetic valve, including folding extended commissure tabs of leaflets of the prosthetic valve over portions of a reinforcing member. In the illustrated example, additional portions of the reinforcing member may be placed over outer surfaces of the folded extended commissure tabs and associated end portions of the reinforcing member may be knotted or otherwise coupled together to form and secure a resulting support structure (e.g., a suture frame) over the extended commissure tabs.

In the illustrated example, commissure tabs 1830a and 1830b of the leaflets of the prosthetic valve are provided with extended tabs 1832a and 1832b, respectively. The extended tabs 1832a and 1832b are folded over primary reinforcing element 1820a portions. Secondary reinforcing element 1820b portions are placed over the outer surfaces of the folded tabs 1832a and 1832b (e.g., where the inner surfaces in a region of the primary reinforcing element portions transitions, due to the folding, to outer surfaces over which the secondary reinforcing element portions are placed), thereby framing the tabs (e.g., forming a suture frame or other support structure). End portions of the secondary reinforcing element 1820b may be knotted or otherwise coupled together to secure the resulting support structure over the tabs.

A post skirt 1840 or other support/buffering material (e.g., a support strip) may be stitched to the folded portion of the commissure tabs 1830a and 1830b, configured to circumvent and attach the commissure to a support post 1810 (e.g., a support structure of a frame of a prosthetic valve). The disclosed commissure configuration can be easily attached to a wide variety of frame posts, including posts of mechanically expandable valves.

According to some embodiments, a single reinforcing member, which can be for example suture (e.g., a braided suture, such as an Ethibond suture), can be used to form both the primary reinforcing element 1820a portions and the secondary reinforcing element 1820b portions. In some examples, the reinforcing member is an Ethibond 3.0 suture. FIG. 19 shows a cross-sectional view of the commissure tabs 1830a and 1830b of FIG. 18, including a single reinforcing member 1820, which is wrapped and/or crossed in different directions between different extensions thereof (exemplary wrap directions shown in dashed lines in Fig. 19). In the example of FIG. 19, the single reinforcing member is continuous, such that primary and secondary reinforcing elements 1820a and 1820b of FIG. 18 correspond to different regions of the same single reinforcing member 1820. In other examples, multiple reinforcing elements may be coupled together or separately coupled to the extended tabs to collectively form a reinforcing member having the primary and secondary reinforcing elements 1820a and 1820b.

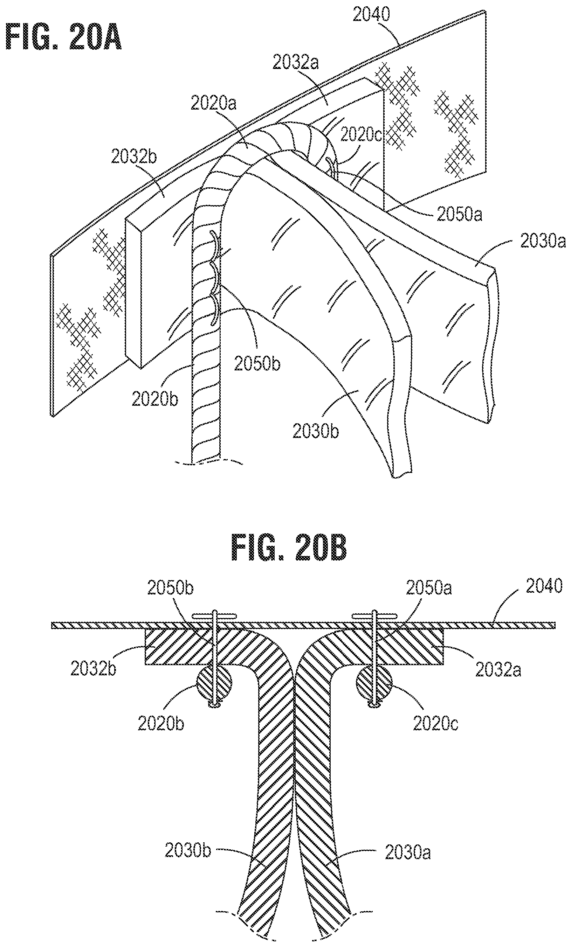

FIG. 20A shows a first stage of an example of assembling a commissure of a prosthetic valve including extended commissure tabs. For example, the configuration shown in FIG. 20A may be a first stage of assembling a commissure as shown in FIGS. 18 and 19.

In the illustrated example, commissure tabs 2030a and 2030b of the leaflets of the prosthetic valve are provided with extended tabs 2032a and 2032b, respectively. In the illustrated first stage, a primary reinforcing element 2020a is placed over the outer surfaces of the extended tabs 2032a and 2032b. For example, side portions 2020b and 2020c of the primary reinforcing element 2020a may be positioned to be in contact with the outer surfaces of the extended tabs 2032b and 2032a, respectively.

In the illustrated example, primary stitches 2050a and 2050b are respectively applied to attach the primary reinforcing element to a post skirt 2040 (or other support/buffering material and/or support strip) through the extended tabs of the leaflet. As described above with respect to the post skirt 1840 of FIG. 18, the post skirt 2040 may be configured to circumvent and attach the commissure to a support post or other support structure of the prosthetic valve (e.g., a support structure of a frame of the prosthetic valve, a frame post, including posts of mechanically expandable valves, etc.).

As shown in more detail in the cross-sectional view of FIG. 20B (e.g., showing a cross-section of the configuration of FIG. 20A), the primary stitch 2050a may pass through, in order, the side portion 2020c of the primary reinforcing element, the extended tab 2032a, and the post skirt 2040. The primary stitch 2050b may pass through, in order, the side portion 2020b of the primary reinforcing element, the extended tab 2032b, and the post skirt 2040. In this way, the primary stitches 2050a and 2050b may take the load of the diastolic pressure forces on the leaflets/commissure tabs.

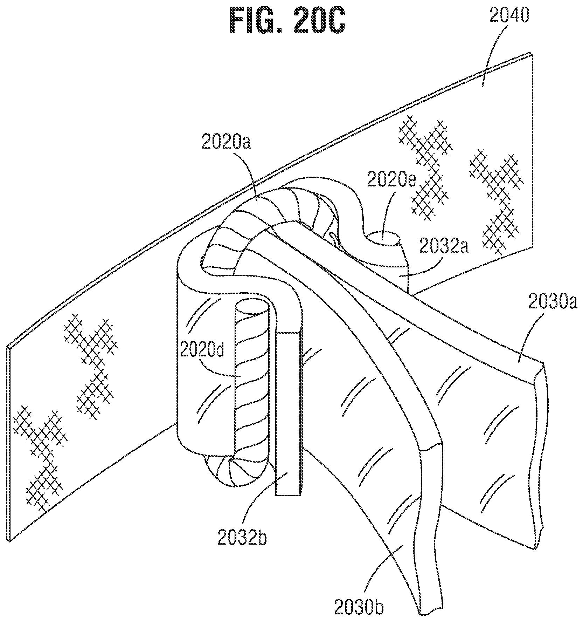

FIG. 20C shows a second stage of example of assembling a commissure of a prosthetic valve including extended commissure tabs, where the extended commissure tabs are folded over at least a portion of the primary reinforcing element. For example, as shown, end portions of the extended tabs 2032a and 2032b are folded (e.g., pulled in a direction back toward the remaining portions of the associated leaflets) around and/or over the respective side portions of the primary reinforcing element (e.g., before caging the associated leaflets in a suture window). At least a portion of the end portions of the extended tabs may be substantially parallel to respective outer surfaces of the associated commissure tabs when folded over the primary reinforcing element.

As further illustrated in FIG. 20C, end portions 2020d and 2020e of the primary reinforcing element 2020a are pulled around an underside of respective folded regions of the extended tabs to align with an outside of the folded regions. For example, end portion 2020d may be positioned against an opposing surface of the extended tab 2032b from the surface that contacts/faces the side portion 2020b (shown in FIG. 20A, not fully visible in the view of FIG. 20C) of the primary reinforcing element. Likewise, end portion 2020e may be positioned against an opposing surface of the extended tab 2032a from the surface that contacts/faces the side portion 2020c (shown in FIG. 20A, not fully visible in the view of FIG. 20C) of the primary reinforcing element.

The end portions 2020d and 2020e may be attached to at least the respective extended tabs 2032b and 2032a (e.g., via secondary sutures). In some examples, the attachment mechanism (e.g., sutures) that attaches the end portion 2020d to the extended tab 2032b may be extended to pass through extended tab 2032a and/or end portion 2020e, thereby also attaching end portions 2020d and 2020e to one another. In additional or alternative examples, the end portions 2020d and 2020e may not be terminated as shown in FIG. 20C and may extend and be knotted or otherwise coupled together to secure the resulting support structure (e.g., formed by the primary reinforcing element) over the extended tabs.

The example illustrated in FIG. 20C shows the extended tabs 2032a and 2032b being reinforced by portions of a single reinforcing element 2020a (which can be for example a suture, such as a braided suture, as described above with respect to reinforcing elements 1820a and 1820b). It is to be understood that the configuration of FIGS. 20A-20C may additionally or alternatively be formed using multiple reinforcing elements that are coupled to one another and/or the extended tabs and other commissure elements in the manner described above for the different portions of the reinforcing element 2020a.

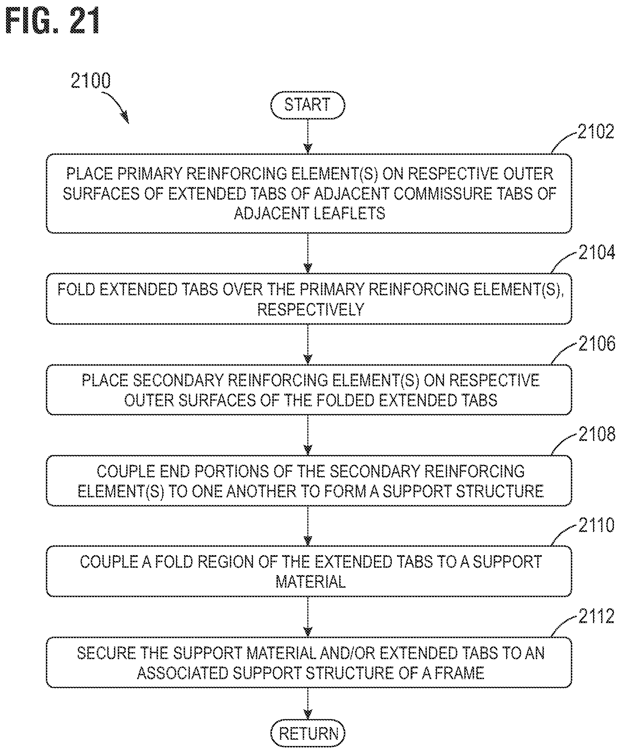

FIG. 21 is a flow chart of an example method 2100 for assembling a commissure of a prosthetic valve using extended commissure tabs of leaflets of the prosthetic valve. For example, at least a portion of method 2100 may be performed to assemble the commissure configurations as illustrated in FIGS. 18, 19, and/or 20A-20C. It is to be understood that one or more elements of method 2100 may be omitted or performed in a different order without departing from the scope of the disclosure. Method 2100 may be iteratively performed to form a plurality of commissures with a plurality of leaflets of a prosthetic valve (e.g., a prosthetic heart valve). Each commissure may be formed by pairing a first commissure tab of a first leaflet with an adjacent, second commissure tab of a second leaflet, then performing at least a portion of method 2100 for the respective pair of commissure tabs. The prosthetic valve may be assembled by mounting each of the resulting commissures to respective support portions (e.g., support struts/posts) of a frame of the prosthetic valve.

At 2102, the method includes placing one or more primary reinforcing elements on respective outer surfaces of extended tabs of adjacent commissure tabs of adjacent leaflets. An example of the placement of the primary reinforcing elements is shown and described above with respect to 1820a of FIG. 18. In the illustrated example, the primary reinforcing elements are portions of a single, continuous primary reinforcing member that extends around edges of the adjacent commissure tabs to contact the outer surfaces of the commissure tabs. The positioning of this example of a primary reinforcing element may be similar to the positioning of the outer reinforcing elements shown and described above with respect to FIGS. 4 and 5.

At 2104, the method includes folding the extended tabs over the one or more primary reinforcing elements, respectively. An example of the folding is shown and described above with respect to FIGS. 18 and 19. As shown, each extended tab is folded over itself (and over a respective primary reinforcing element placed on its outer surface) to create a loop in which the respective primary reinforcing element is positioned. As a result of the folding described above, the primary reinforcing elements are positioned such that they are each adjacent to a respective inner surface of the folded extended commissure tab (e.g., the region of the extended commissure tab that extends from the fold in the extended commissure tab toward an end portion of the extended commissure tab).

At 2106, the method includes placing one or more secondary reinforcing elements on respective outer surfaces of the folded extended tabs. An example of the placement of the secondary reinforcing elements is shown and described above with respect to FIG. 18. In the illustrated example, the secondary reinforcing elements are portions of a single, continuous secondary reinforcing member that extends around edges of the adjacent commissure tabs to contact the outer surfaces of the folded extended tabs. It is to be understood that in other examples, multiple secondary reinforcing elements may be used to contact the outer surfaces of the folded extended tabs. As shown, the outer surfaces of the folded extended tabs extend from the inner surfaces of the extended tabs due to the folding of the extended tabs (e.g., at a fold region of the extended tabs, the inner surfaces of the extended tabs become outer surfaces of the folded extended tabs). In this way, for each extended commissure tab, the respective primary and secondary reinforcing elements are positioned adjacent opposite surfaces of the folded extended tab from one another.

At 2108, the method includes coupling end portions of the one or more secondary reinforcing elements to one another to form a support structure. For example, the end portions of the secondary reinforcing elements may be coupled to one another across an edge of the extended tabs, at an outer surface of the folded extended tabs, and/or within an associated loop formed by folding the extended tabs over themselves as described at 2104.

At 2110, the method includes coupling a fold region of the extended tabs to a support material, such as a support strip and/or post skirt. For example, the fold region of the extended tabs may include portions of the extended tabs that are folded over the primary reinforcing elements as described at 2104. Any suitable mechanism may be used to couple the extended tabs (e.g., in the fold region) to the support material.

At 2112, the method includes securing the support material and/or the extended tabs to an associated support structure of a frame of the prosthetic valve (e.g., support structure 1810 of FIG. 18). In some examples, the mechanism used to couple the fold region of the extended tabs to the support material may also be used at least in part to secure the support material and/or the extended tabs to the associated support structure of the frame. In additional or alternative examples, the primary and/or secondary reinforcing elements may be directly or indirectly coupled to the support material and/or to a coupling mechanism used to secure the support material and/or extended tabs to the support structure. Any suitable mechanism for securing the support material and/or the extended tabs to the support structure may be used. For example, direct coupling (e.g., where portions of the support material and/or the extended tabs are directly sutured, adhered, or otherwise coupled and/or in contact with the support structure and/or with one another), indirect coupling via a single suture, indirect coupling via multiple sutures, and/or any other suitable coupling mechanism. In some examples, direct attachment of portions of the support material to one another (e.g., after wrapping the support material around the support structure of the frame) may be reinforced by a further suture or other coupling mechanism applied to a region where the portions of the support material are coupled to one another (e.g., stitching a suture through the portions of the support material in the region of direct attachment). Securing the commissure to the support portion of the frame may utilize any suitable coupling mechanisms, such as stitching (e.g., shoelace stitching), knotting/tying (e.g., square knotting), adhering, sintering, and/or any other mechanism or combination of mechanisms.

### Additional Examples of the Disclosed Technology

In view of the above described implementations of the disclosed subject matter, this application discloses the additional examples enumerated below. It should be noted that one feature of an example in isolation or more than one feature of the example taken in combination and, optionally, in combination with one or more features of one or more further examples are further examples also falling within the disclosure of this application.

Example 1. A method of assembling a prosthetic heart valve including a plurality of leaflets, the method comprising: forming a plurality of commissures with the plurality of leaflets, wherein each commissure is formed by pairing a first commissure tab of a first leaflet with an adjacent, second commissure tab of a second leaflet, folding a support strip over an inner reinforcing element, positioning the folded support strip between the first commissure tab and the second commissure tab, and attaching first and second outer reinforcing elements to the first and second commissure tabs, respectively, via stitching that extends through the first outer reinforcing element, the first leaflet, the folded support strip, the second leaflet, and the second outer reinforcing element, the stitching extending over at least a portion of the first reinforcing element opposite a fold region of the support strip, and, for each commissure, securing end portions of the support strip to a respective support portion of a frame of the prosthetic heart valve.

Example 2. The method of any example herein, particularly example 1, wherein securing the end portions of the support strip to the respective support portion of the frame comprises stitching a respective suture to each end portion of the support strip, extending the respective sutures around the support portion of the frame, and attaching the respective sutures to one another.

Example 3. The method of any example herein, particularly any one of examples 1-2, wherein securing the end portions of the support strip to the respective support portion of the frame comprises extending the end portions of the folded support strip around the support portion of the frame and attaching the end portions of the folded support strip directly to one another.

Example 4. The method of any example herein, particularly example 3, further comprising reinforcing a region of direct attachment of the end portions of the support strip by stitching a suture through the end portions of the support strip in the region of direct attachment.

Example 5. The method of any example herein, particularly any one of examples 1-4, wherein the end portions of the support strip extend past end portions of the first and second commissure tabs, respectively, and wherein securing the end portions of the support strip to the respective support portion of the frame comprises folding the end portions of the support strip around the end portions of the first and second commissure tabs, respectively, stitching the end portions of the support strip to the end portions of the first and second commissure tabs, respectively, via at least one suture, and coupling the end portions of the support strip via the at least one suture.

Example 6. The method of any example herein, particularly example 5, wherein the at least one suture comprises a single suture that passes through both end portions of the support strip, and wherein coupling the end portions of the support strip via the at least one suture comprises extending end portions of the single suture around the support structure in opposing directions from one another and coupling the end portions of the single suture.

Example 7. The method of any example herein, particularly example 5, wherein the at least one suture comprises a first suture that passes through a first end portion of the support strip and a second suture that passes through the second end portion of the folded support strip, and wherein coupling the end portions of the support strip via the at least one suture comprises extending the first and second sutures around the support structure and coupling the first and second sutures.

Example 8. The method of any example herein, particularly any one of examples 1-7, wherein the end portions of the support strip are coupled tying a suture around the support structure.

Example 9. The method of any example herein, particularly any one of examples 1-8, wherein securing the end portions of the support strip to the respective support portion of the frame comprises at least partially folding the first and second commissure tabs around the first and second outer reinforcing elements, respectively, to create a fold region of the stitching extending across the inner reinforcing element, and wherein the inner reinforcing element is positioned between the fold region of the stitching and the fold region of the support strip.

Example 10. The method of any example herein, particularly example 9, wherein at least partially folding the first and second commissure tabs comprises folding the first and second commissure tabs until the first and second outer reinforcing elements are aligned with the inner reinforcing element.

Example 11. The method of any example herein, particularly any one of examples 1-10, further comprising coupling the inner reinforcing element to the support strip.

Example 12. The method of any example herein, particularly example 11, wherein coupling the inner reinforcing element to the support strip comprises attaching the inner reinforcing element to the support strip using a suture that forms stitches passing through the support strip and the inner reinforcing element.

Example 13. The method of any example herein, particularly any one of examples 11-12, wherein coupling the inner reinforcing element to the support strip comprises coupling the inner reinforcing element to the support strip along a vertical centerline of the support strip.

Example 14. The method of any example herein, particularly any one of examples 11-13, wherein coupling the inner reinforcing element to the support strip comprises adhering, melting, and/or sintering the inner reinforcing element to the support strip.

Example 15. The method of any example herein, particularly any one of examples 11-14, wherein the support strip includes one or more stitching lines or markings visually indicating locations for placing sutures during assembly, and wherein attaching the first and second outer reinforcing elements to the first and second commissure tabs comprises aligning each of the first and second outer reinforcing elements to a respective subset of the one or more stitching lines or markings.

Example 16. The method of any example herein, particularly any one of examples 1-14, wherein attaching the first and second outer reinforcing elements to the first and second commissure tabs comprises aligning the first and second outer reinforcing elements with the inner reinforcing element.

Example 17. A prosthetic heart valve comprising: an annular frame comprising a plurality of commissure support portions, and a plurality of leaflets, each leaflet having a commissure tab that is coupled to an adjacent commissure tab of another leaflet via a folded support strip to form a commissure of an associated commissure tab pair, wherein, for each commissure tab pair: the folded support strip is disposed between respective inner surfaces of the commissure tabs in the commissure tab pair, an inner reinforcing element is disposed in a fold region of the folded support strip, first and second outer reinforcing elements are positioned adjacent respective outer surfaces of the commissure tabs in the commissure tab pair, a reinforcing suture passes through, in order: the first outer reinforcing element, a first commissure tab of the commissure tab pair, a first section of the folded support strip, a second section of the folded support strip, a second commissure tab of the commissure tab pair, and a second outer reinforcing element, and the inner reinforcing element is positioned between the fold region of the folded support strip and the reinforcing suture, and wherein each commissure is secured to a corresponding commissure support portion of the plurality of commissure support portions.

Example 18. The prosthetic heart valve of any example herein, particularly example 17, wherein each commissure is secured to the corresponding commissure support portion via at least one securing suture extending from the folded support strip.

Example 19. The prosthetic heart valve of any example herein, particularly example 18, wherein, for each commissure tab pair, the at least one securing suture includes a first securing suture coupled to and extending from a first end portion of the folded support strip and a second securing suture coupled to and extending from a second end portion of the folded support strip, the first end portion being opposite of the second end portion, and the first securing suture being coupled to the second securing suture.

Example 20. The prosthetic heart valve of any example herein, particularly example 19, wherein, for each commissure tab pair, the first end portion of the folded support strip is folded over an end portion of the first commissure tab and the second end portion of the folded support strip is folded over an end portion of the second commissure tab.

Example 21. The prosthetic heart valve of any example herein, particularly example 20, wherein, for each commissure tab pair, the first securing suture and the second securing suture couple the first end portion of the folded support strip to the first commissure tab and the second end portion of the folded support strip to the second commissure tab, respectively.

Example 22. The prosthetic heart valve of any example herein, particularly example 18, wherein, for each commissure tab pair, a first end portion of the folded support strip extends past an end portion of the first commissure tab at least partially around the corresponding commissure support portion in a first radial direction, wherein a second end portion of the folded support strip extends past an end portion of the second commissure tab at least partially around the corresponding commissure support portion in a second radial direction that is opposite the first radial direction, and wherein the at least one securing suture comprises a single securing suture attaching the first end portion of the folded support strip to the second end portion of the folded support strip.

Example 23. The prosthetic heart valve of any example herein, particularly example 20 or 22, further comprising, for each commissure tab pair, a first supplemental suture that couples the folded support strip to the first commissure tab and a second supplemental suture that couples the folded support strip to the second commissure tab.

Example 24. The prosthetic heart valve of any example herein, particularly example 17, wherein, for each commissure tab pair, the first commissure tab and the second commissure tab are partially folded in opposing directions to one another, the first outer reinforcing element is positioned in a fold region of the first commissure tab adjacent an outer surface of the first commissure tab, the second outer reinforcing element is positioned in a fold region of the second commissure tab adjacent an outer surface of the second commissure tab, and the fold region of the folded support strip and the inner reinforcing element are positioned between an inner surface of the first commissure tab and an inner surface of the second commissure tab.

Example 25. The prosthetic heart valve of any example herein, particularly any one of examples 17-24, wherein, for each commissure tab pair, the corresponding commissure support portion comprises a component of an actuator that produces radial expansion of the prosthetic heart valve.

Example 26. The prosthetic heart valve of any example herein, particularly any one of examples 17-25, wherein the inner reinforcing element is coupled to the support strip.

Example 27. The prosthetic heart valve of any example herein, particularly example 26, wherein the inner reinforcing element is attached to the support strip using a suture that forms stitches passing through the support strip and the inner reinforcing element.

Example 28. The prosthetic heart valve of any example herein, particularly any one of examples 26-27, wherein the inner reinforcing element is coupled to the support strip along a vertical centerline of the support strip.

Example 29. The prosthetic heart valve of any example herein, particularly any one of examples 26-28, wherein the inner reinforcing element is adhered, melted, and/or sintered to the support strip.

Example 30. The prosthetic heart valve of any example herein, particularly any one of examples 17-29, wherein the support strip includes material that is more resilient to tearing and/or deforming than material used for forming the plurality of leaflets and/or the commissure tabs.

Example 31. The prosthetic heart valve of any example herein, particularly any one of examples 17-30, wherein the support strip comprises a polyethylene terephthalate (PET) fabric.

Example 32. The prosthetic heart valve of any example herein, particularly any one of examples 17-31, wherein the support strip is continuous, with a uniform distribution of material.

Example 33. The prosthetic heart valve of any example herein, particularly any one of examples 17-32, wherein the support strip is longer in one dimension than another.

Example 34. The prosthetic heart valve of any example herein, particularly any one of examples 17-33, wherein the support strip has a thickness that is smaller than a diameter of the inner reinforcing element.

Example 35. The prosthetic heart valve of any example herein, particularly any one of examples 17-34, wherein the support strip includes one or more stitching lines or markings visually indicating locations for placing sutures during assembly.

Example 36. The prosthetic heart valve of any example herein, particularly example 35, wherein the stitching lines or markings are formed with a plurality of pre-formed apertures or openings for receiving sutures, and the apertures or openings extend at least partially through the support strip.

Example 37. The prosthetic heart valve of any example herein, particularly any one of examples 17-36, wherein the inner reinforcing element comprises a string, cord, or suture that has a larger diameter than the reinforcing suture.

Example 38. The prosthetic heart valve of any example herein, particularly any one of examples 17-36, wherein the inner reinforcing element comprises a folded strip of fabric.

Example 39. The prosthetic heart valve of any example herein, particularly any one of examples 17-36, wherein the inner reinforcing element comprises a wire or bar formed from a metal and/or a polymer.

Example 40. The prosthetic heart valve of any example herein, particularly any one of examples 17-39, wherein the first and second outer reinforcing elements form different sections of a single continuous outer reinforcing member.

Example 41. The prosthetic heart valve of any example herein, particularly example 40, wherein the outer reinforcing member is a single member folded into a U-shape.

Example 42. The prosthetic heart valve of any example herein, particularly any one of examples 17-39, wherein the first and second outer reinforcing elements are discrete and spatially separated from one another.

Example 43. The prosthetic heart valve of any example herein, particularly any one of examples 17-42, wherein each of the first and second outer reinforcing elements comprises a string, cord, or suture that has a larger diameter than the reinforcing suture.

Example 44. The prosthetic heart valve of any example herein, particularly any one of examples 17-42, wherein each of the first and second outer reinforcing elements comprises a folded strip of fabric.

Example 45. The prosthetic heart valve of any example herein, particularly any one of examples 17-42, wherein each of the first and second outer reinforcing elements comprises a wire or bar formed from a metal and/or a polymer.

Example 46. A method of assembling a commissure for a leaflet pair of a prosthetic valve, the method comprising: folding a support strip over an inner reinforcing element to form a first folded portion of the support strip extending from a fold region of the support strip to a first end portion and a second folded portion of the support strip extending from the fold region of the support strip to a second end portion opposite the first end portion, positioning the folded support strip between inner surfaces of adjacent ends of a first commissure tab of a first leaflet of the leaflet pair and a second commissure tab of a second leaflet of the leaflet pair, folding an outer reinforcing member into a U-shape to form a first outer reinforcing element and a second outer reinforcing element, placing the first outer reinforcing element against an outer surface of the first commissure tab and the second outer reinforcing element against an outer surface of the second commissure, forming, via a suture, one or more stitches that pass through, in order, the first outer reinforcing element, the first commissure tab, the first folded portion of the support strip, the second folded portion of the support strip, the second commissure tab, and the second outer reinforcing element, so that the inner reinforcing element is positioned between the fold region of the support strip and the suture, and coupling the first end portion of the folded support strip to the second end portion of the folded support strip around a support portion of a frame of the prosthetic valve to secure the commissure to the support portion of the frame.

Example 47. The method of any example herein, particularly example 46, further comprising reinforcing a region of direct attachment of the first and second end portions of the support strip by stitching a suture through the first and second end portions of the support strip in the region of direct attachment.

Example 48. The method of any example herein, particularly any one of examples 46-47, further comprising coupling the inner reinforcing element to the support strip.

Example 49. The method of any example herein, particularly example 48, wherein coupling the inner reinforcing element to the support strip comprises attaching the inner reinforcing element to the support strip using a suture that forms stitches passing through the support strip and the inner reinforcing element.

Example 50. The method of any example herein, particularly any one of examples 48-49, wherein the inner reinforcing element is coupled to the support strip along a vertical centerline of the support strip.

Example 51. A method of assembling a prosthetic heart valve comprising a plurality of leaflets, the method comprising: forming a plurality of commissures with the plurality of leaflets, wherein each commissure is formed by pairing a first commissure tab of a first leaflet with an adjacent, second commissure tab of a second leaflet, folding a first portion of a support strip over a first edge reinforcing element, folding a second portion of the support strip over a second edge reinforcing element, coupling an end portion of the first portion of the support strip to an end portion of the second portion of the support strip to form a reinforced region of the support strip, positioning the support strip along respective inner surfaces of the first commissure tab and the second commissure tab, coupling first and second outer reinforcing elements to the first and second commissure tabs, respectively, via stitching that extends through the first outer reinforcing element, the first commissure tab, the folded support strip, the second commissure tab, and the second outer reinforcing element, the stitching extending over the reinforced region of the support strip, and for each commissure, securing the support strip to a respective support portion of a frame of the prosthetic heart valve.

Example 52. The method of any example herein, particularly example 51, wherein the reinforced region is positioned at substantially a vertical centerline of the support strip.

Example 53. The method of any example herein, particularly any one of examples 51-52, further comprising respectively folding the end portions of the first and second portions of the support strip over stitches used for coupling the end portions to one another.

Example 54. The method of any example herein, particularly any one of examples 51-53, wherein securing the support strip to the respective support portion of the frame comprises stitching a respective suture to folded portions of the support strip that are folded around the first and second edge reinforcing elements, respectively, extending the respective sutures around the support portion of the frame, and attaching the respective sutures to one another.

Example 55. The method of any example herein, particularly any one of examples 51-53, wherein securing the support strip to the respective support portion of the frame comprises extending the folded portions of the support strip that are folded around the first and second edge reinforcing elements, respectively, around the support portion of the frame and directly attaching the folded portions of the support strip and/or the first and second edge reinforcing elements to one another.

Example 56. The method of any example herein, particularly example 55, further comprising reinforcing a region of direct attachment of the folded regions of the support strip by stitching a suture through the folded regions of the support strip in the region of direct attachment.

Example 57. The method of any example herein, particularly any one of examples 51-53, wherein folded regions of the support strip that are folded around the first and second edge reinforcing elements, respectively, extend past end portions of the first and second commissure tabs, respectively, and wherein securing the support strip to the respective support portion of the frame comprises folding the folded regions of the support strip around the end portions of the first and second commissure tabs, respectively, stitching the folded regions of the support strip and/or the first and second edge reinforcing elements to the end portions of the first and second commissure tabs, respectively, via at least one suture, and coupling the folded regions of the support strip via the at least one suture.

Example 58. The method of any example herein, particularly example 57, wherein the at least one suture comprises a single suture that passes through both folded regions of the support strip, and wherein coupling the folded regions of the support strip via the at least one suture comprises extending end portions of the single suture around the support structure in opposing directions from one another and coupling the end portions of the single suture.

Example 59. The method of any example herein, particularly example 57, wherein the at least one suture comprises a first suture that passes through a first folded region of the support strip and a second suture that passes through a second folded region of the folded support strip, and wherein coupling the folded regions of the support strip via the at least one suture comprises extending the first and second sutures around the support structure and coupling the first and second sutures.

Example 60. The method of any example herein, particularly any one of examples 51-53, wherein folded regions of the support strip that are folded around the first and second edge reinforcing elements, respectively, are coupled via tying a suture around the support structure.

Example 61. The method of any example herein, particularly any one of examples 51-60, wherein securing the support strip to the respective support portion of the frame comprises at least partially folding, at a fold region of the commissure tabs, the first and second commissure tabs around the first and second outer reinforcing elements, respectively, to create a fold region of the stitching extending across the reinforcing region of the support strip, and wherein the reinforcing region of the support strip is positioned between the fold region of the stitching and a fold region of the commissure tabs.

Example 62. A prosthetic heart valve comprising: an annular frame comprising a plurality of commissure support portions, and a plurality of leaflets, each leaflet having a commissure tab that is coupled to an adjacent commissure tab of another leaflet via a folded support strip to form a commissure of an associated commissure tab pair, and each commissure tab pair is assembled and/or coupled to the annular frame according to the method of any example herein, particularly any one of examples 51-61.

Example 63. A method of assembling a prosthetic heart valve comprising a plurality of leaflets, the method comprising: forming a plurality of commissures with the plurality of leaflets, wherein each commissure is formed by pairing a first commissure tab of a first leaflet with an adjacent, second commissure tab of a second leaflet, the first commissure tab comprising a first extended tab and the second commissure tab comprising a second extended tab, folding the first and second extended tabs over first and second portions of a primary reinforcing element, respectively, placing first and second portions of a secondary reinforcing element on respective outer surfaces of the folded first and second extended tabs, and coupling end portions of the first and second portions of the secondary reinforcing element to one another, and, for each commissure, coupling a fold region of the first and second extended tabs to a support material and securing the support material and/or the first and second extended tabs to an associated support structure of a frame of the prosthetic heart valve.

Example 64. The method of any example herein, particularly example 63, wherein the primary reinforcing element and the secondary reinforcing element are different portions of a same continuous reinforcing member.

Example 65. The method of any example herein, particularly example 63, wherein the first and second portions of the primary reinforcing element are different portions of a same continuous primary reinforcing member.

Example 66. The method of any example herein, particularly example 63, wherein the first and second portions of the secondary reinforcing element are different portions of a same continuous secondary reinforcing member.

Example 67. A prosthetic heart valve comprising: an annular frame comprising a plurality of commissure support portions, and a plurality of leaflets, each leaflet having a commissure tab that is coupled to an adjacent commissure tab of another leaflet via a folded support strip to form a commissure of an associated commissure tab pair, and each commissure tab pair is assembled and/or coupled to the annular frame according to the method of any example herein, particularly any one of examples 63-66.
Examples are set out in the following list of numbered clauses:
1. A method of assembling a prosthetic heart valve comprising a plurality of leaflets, the method comprising:
   forming a plurality of commissures with the plurality of leaflets, wherein each commissure is formed by:
      pairing a first commissure tab of a first leaflet with an adjacent, second commissure tab of a second leaflet,
      folding a support strip over an inner reinforcing element,
      positioning the folded support strip between the first commissure tab and the second commissure tab, and
      attaching first and second outer reinforcing elements to the first and second commissure tabs, respectively, via stitching that extends through the first outer reinforcing element, the first leaflet, the folded support strip, the second leaflet, and the second outer reinforcing element, the stitching extending over at least a portion of the first reinforcing element opposite a fold region of the support strip; and
   for each commissure, securing end portions of the support strip to a respective support portion of a frame of the prosthetic heart valve.
2. The method of clause 1, wherein securing the end portions of the support strip to the respective support portion of the frame comprises stitching a respective suture to each end portion of the support strip, extending the respective sutures around the support portion of the frame, and attaching the respective sutures to one another.
3. The method of clause 1, wherein securing the end portions of the support strip to the respective support portion of the frame comprises extending the end portions of the folded support strip around the respective support portion of the frame and attaching the end portions of the folded support strip directly to one another.
4. The method of clause 3, further comprising reinforcing a region of direct attachment of the end portions of the support strip by stitching a suture through the end portions of the support strip in the region of direct attachment.
5. The method of clause 1, wherein the end portions of the support strip extend past end portions of the first and second commissure tabs, respectively, and wherein securing the end portions of the support strip to the respective support portion of the frame comprises folding the end portions of the support strip around the end portions of the first and second commissure tabs, respectively, stitching the end portions of the support strip to the end portions of the first and second commissure tabs, respectively, via at least one suture, and coupling the end portions of the support strip via the at least one suture.
6. The method of clause 5, wherein the at least one suture comprises a single suture that passes through both end portions of the support strip, and wherein coupling the end portions of the support strip via the at least one suture comprises extending end portions of the single suture around the respective support portion of the frame in opposing directions from one another and coupling the end portions of the single suture.
7. The method of clause 5, wherein the at least one suture comprises a first suture that passes through a first end portion of the support strip and a second suture that passes through the second end portion of the folded support strip, and wherein coupling the end portions of the support strip via the at least one suture comprises extending the first and second sutures around the respective support portion of the frame and coupling the first and second sutures.
8. The method of clause 1, wherein the end portions of the support strip are coupled via tying a suture around the respective support portion of the frame.
9. The method of any one of clauses 1-8, wherein securing the end portions of the support strip to the respective support portion of the frame comprises at least partially folding the first and second commissure tabs around the first and second outer reinforcing elements, respectively, to create a fold region of the stitching extending across the inner reinforcing element, and wherein the inner reinforcing element is positioned between the fold region of the stitching and the fold region of the support strip.
10. The method of clause 9, wherein at least partially folding the first and second commissure tabs comprises folding the first and second commissure tabs until the first and second outer reinforcing elements are aligned with the inner reinforcing element.
11. A prosthetic heart valve comprising:
   an annular frame comprising a plurality of commissure support portions; and
   a plurality of leaflets, each leaflet having a commissure tab that is coupled to an adjacent commissure tab of another leaflet via a folded support strip to form a commissure of an associated commissure tab pair,
   wherein, for each commissure tab pair:
      the folded support strip is disposed between respective inner surfaces of the commissure tabs in the commissure tab pair, an inner reinforcing element is disposed in a fold region of the folded support strip, first and second outer reinforcing elements are positioned adjacent respective outer surfaces of the commissure tabs in the commissure tab pair, a reinforcing suture passes through, in order: the first outer reinforcing element, a first commissure tab of the commissure tab pair, a first section of the folded support strip, a second section of the folded support strip, a second commissure tab of the commissure tab pair, and a second outer reinforcing element, and the inner reinforcing element is positioned between the fold region of the folded support strip and the reinforcing suture, and
   wherein each commissure is secured to a corresponding commissure support portion of the plurality of commissure support portions.
12. The prosthetic heart valve of clause 11, wherein each commissure is secured to the corresponding commissure support portion via at least one securing suture extending from the folded support strip.
13. The prosthetic heart valve of clause 12, wherein, for each commissure tab pair, the at least one securing suture includes a first securing suture coupled to and extending from a first end portion of the folded support strip and a second securing suture coupled to and extending from a second end portion of the folded support strip, the first end portion being opposite of the second end portion, and the first securing suture being coupled to the second securing suture.
14. The prosthetic heart valve of clause 13, wherein, for each commissure tab pair, the first end portion of the folded support strip is folded over an end portion of the first commissure tab and the second end portion of the folded support strip is folded over an end portion of the second commissure tab.
15. The prosthetic heart valve of clause 14, wherein, for each commissure tab pair, the first securing suture and the second securing suture couple the first end portion of the folded support strip to the first commissure tab and the second end portion of the folded support strip to the second commissure tab, respectively.
16. The prosthetic heart valve of clause 12, wherein, for each commissure tab pair, a first end portion of the folded support strip extends past an end portion of the first commissure tab at least partially around the corresponding commissure support portion in a first radial direction, wherein a second end portion of the folded support strip extends past an end portion of the second commissure tab at least partially around the corresponding commissure support portion in a second radial direction that is opposite the first radial direction, and wherein the at least one securing suture comprises a single securing suture attaching the first end portion of the folded support strip to the second end portion of the folded support strip.
17. The prosthetic heart valve of clause 14, further comprising, for each commissure tab pair, a first supplemental suture that couples the folded support strip to the first commissure tab and a second supplemental suture that couples the folded support strip to the second commissure tab.
18. The prosthetic heart valve of clause 11, wherein, for each commissure tab pair, the first commissure tab and the second commissure tab are partially folded in opposing directions to one another, the first outer reinforcing element is positioned in a fold region of the first commissure tab adjacent an outer surface of the first commissure tab, the second outer reinforcing element is positioned in a fold region of the second commissure tab adjacent an outer surface of the second commissure tab, and the fold region of the folded support strip and the inner reinforcing element are positioned between an inner surface of the first commissure tab and an inner surface of the second commissure tab.
19. The prosthetic heart valve of any one of clauses 11-18, wherein, for each commissure tab pair, the corresponding commissure support portion comprises a component of an actuator that produces radial expansion of the prosthetic heart valve.
20. A method of assembling a commissure for a leaflet pair of a prosthetic valve, the method comprising:
   folding a support strip over an inner reinforcing element to form a first folded portion of the support strip extending from a fold region of the support strip to a first end portion and a second folded portion of the support strip extending from the fold region of the support strip to a second end portion opposite the first end portion;
   positioning the folded support strip between inner surfaces of adjacent ends of a first commissure tab of a first leaflet of the leaflet pair and a second commissure tab of a second leaflet of the leaflet pair;
   folding an outer reinforcing member into a U-shape to form a first outer reinforcing element and a second outer reinforcing element;
   placing the first outer reinforcing element against an outer surface of the first commissure tab and the second outer reinforcing element against an outer surface of the second commissure;
   forming, via a suture, one or more stitches that pass through, in order, the first outer reinforcing element, the first commissure tab, the first folded portion of the support strip, the second folded portion of the support strip, the second commissure tab, and the second outer reinforcing element, so that the inner reinforcing element is positioned between the fold region of the support strip and the suture; and
   coupling the first end portion of the folded support strip to the second end portion of the folded support strip around a support portion of a frame of the prosthetic valve to secure the commissure to the support portion of the frame.

## Claims

1. A method of assembling a prosthetic heart valve (10) comprising a plurality of leaflets, the method comprising:
forming a plurality of commissures (24) with the plurality of leaflets, wherein each commissure is formed by pairing a first commissure tab (1430a) of a first leaflet with an adjacent, second commissure tab (1430b) of a second leaflet;
at a first edge reinforcing region of a support strip (1412), folding the support strip over itself to create a double layer, wherein the first edge reinforcing region (1614) is positioned between a first portion (1414a) of the support strip (1412) and a third portion (1414c) of the support strip (1412) and wherein the support strip (1412) is wider at the first edge reinforcing region (1614) of the support strip (1412) than in the first portion (1414a) of the support strip (1412) and a third portion (1414c) of the support strip (1412);
folding (1702) the support strip (1412) widthwise at the first edge reinforcing region (1614) to create a first edge fold region;
at a second edge reinforcing region of a support strip (1412), folding the support strip over itself to create a double layer, wherein the second edge reinforcing region (1614) is positioned between a second portion (1414b) of the support strip (1412) and a fourth portion (1414d) of the support strip (1412) and wherein the support strip (1412) is wider at the second edge reinforcing region (1614) of the support strip (1412) than in the second portion (1414b) of the support strip (1412) and a fourth portion (1414c) of the support strip (1412);folding (1704) the support strip widthwise at the second edge reinforcing region (1614) to create a second edge fold region;
coupling (1706) an end portion (1414e) of the third portion (1414c) of the support strip to an end portion (1414f) of the fourth portion (1414d) of the support strip to form a reinforced region of the support strip;
positioning (1710) the support strip along respective inner surfaces of the first commissure tab and the second commissure tab;
coupling (1714) first and second outer reinforcing elements (1424a, 1424b) to the first and second commissure tabs, respectively, via stitching (1464) that extends through the first outer reinforcing element, the first commissure tab, the folded support strip, the second commissure tab, and the second outer reinforcing element, the stitching extending over the reinforced region of the support strip; and
for each commissure, securing (1716) the support strip to a respective support portion (1450) of a frame of the prosthetic heart valve (10).

2. The method of claim 1, wherein:
a) folding the support strip over itself to create the double layer at the first edge reinforcing region of the support strip (1412) comprises folding a first end portion and a second end portion of the first edge reinforcing region (1614) of the support strip (1412) towards one another, and wherein, in the first end portion of the first edge reinforcing region (1614) prior to folding of the first end portion, a height of the support strip (1412) in the axial direction of the valve in which the support strip is included is increased on an upper region of the support strip compared to the height of the support strip (1412) in the first portion (1414a) and the third portion (1414c), and wherein, in the second end portion of the first edge reinforcing region (1614) prior to folding of the second end portion, a height of the support strip (1412) in the axial direction of the valve in which the support strip is included is increased on a lower region of the support strip compared to the height of the support strip (1412) in the first portion (1414a) and the third portion (1414c); and/or
b) folding the support strip over itself to create the double layer at the second edge reinforcing region of the support strip (1412) comprises folding a first end portion and a second end portion of the second edge reinforcing region (1614) of the support strip (1412) towards one another, and wherein, in the first end portion of the second edge reinforcing region (1614) prior to folding of the first end portion, a height of the support strip (1412) in the axial direction of the valve in which the support strip is included is increased on the upper region of the support strip compared to the height of the support strip (1412) in the second portion (1414b) and the fourth portion (1414d), and wherein, in the second end portion of the second edge reinforcing region (1614) prior to folding of the second end portion, a height of the support strip (1412) in the axial direction of the valve in which the support strip is included is increased on the lower region of the support strip compared to the height of the support strip (1412) in the second portion (1414b) and the fourth portion (1414d).

3. The method of claim 1 or claim 2, wherein the first and the second edge reinforcing region (1614) each include a plurality of stitching lines (1616), optionally wherein the stitching lines comprise:
a) an external marking visually indicating suturing locations for a user assembling the commissure; and/or
b) a plurality of apertures or openings through which a needle carrying sutures can pass.

4. The method of claim 3, insofar as it depends on claim 2, wherein folding the first end portion and the second end portion of the first edge reinforcing region (1614) towards one another and folding the first end portion and the second end portion of the second edge reinforcing region (1614) towards one another additionally comprises, for each end portion of each edge reinforcing region (1614), aligning one or more external markings, apertures and/or openings in an outer row of the respective end portion of the edge reinforcing region with one or more corresponding external markings, apertures and/or openings in an inner row of the respective end portion of the edge reinforcing region, such that each end portion at least partially overlaps itself when in the folded state.

5. The method of any preceding claim, wherein the reinforced region is positioned at substantially a vertical centerline of the support strip.

6. The method of any preceding claim, further comprising respectively folding (1708) the end portions (1414e, 1414f) of the third and fourth portions (1414c, 1414d) of the support strip over stitches (1468) used for coupling the end portions to one another.

7. The method of any preceding claim, wherein securing the support strip to the respective support portion of the frame comprises:
a) stitching a respective suture (1466) to folded portions of the support strip that are folded at the first and second edge fold regions, respectively, extending the respective sutures around the support portion of the frame, and attaching the respective sutures to one another;
b) extending the folded portions of the support strip that are folded at the first and second edge fold regions, respectively, around the support portion of the frame and directly attaching the folded portions of the support strip to one another, optionally further comprising reinforcing a region of direct attachment of the folded regions of the support strip by stitching a suture through the folded regions of the support strip in the region of direct attachment; or
c) at least partially folding, at a fold region of the commissure tabs, the first and second commissure tabs around the first and second outer reinforcing elements, respectively, to create a fold region of the stitching extending across the reinforcing region of the support strip, and wherein the reinforcing region of the support strip is positioned between the fold region of the stitching and a fold region of the commissure tabs.

8. The method of any of claims 1 to 6, wherein folded regions of the support strip that are folded at the first and second edge fold regions, respectively, extend past end portions of the first and second commissure tabs, respectively, and wherein securing the support strip to the respective support portion of the frame comprises folding the folded regions of the support strip around the end portions of the first and second commissure tabs, respectively, stitching the folded regions of the support strip to the end portions of the first and second commissure tabs, respectively, via at least one suture, and coupling the folded regions of the support strip via the at least one suture,
optionally wherein the at least one suture comprises:
a) a single suture that passes through both folded regions of the support strip, and wherein coupling the folded regions of the support strip via the at least one suture comprises extending end portions of the single suture around the support structure in opposing directions from one another and coupling the end portions of the single suture; or
b) a first suture that passes through a first folded region of the support strip and a second suture that passes through a second folded region of the folded support strip, and wherein coupling the folded regions of the support strip via the at least one suture comprises extending the first and second sutures around the support structure and coupling the first and second sutures.

9. The method of any of claim 1 to 6, wherein folded regions of the support strip that are folded at the first and second edge fold regions, respectively, are coupled via tying a suture around the support structure.

10. A prosthetic heart valve (10) comprising:
an annular frame comprising a plurality of commissure support portions (1450); and
a plurality of leaflets (22), each leaflet having a commissure tab (1430) that is coupled to an adjacent commissure tab of another leaflet via a folded support strip to form a commissure (24) of an associated commissure tab pair;
wherein, for each commissure tab pair:
the support strip (1412) is positioned along respective inner surfaces of the first commissure tab (1430a) and the second commissure tab (1430b);
at a first edge reinforcing region of a support strip (1412), the support strip is folded over itself to create a double layer, wherein the first edge reinforcing region (1614) is positioned between a first portion (1414a) of the support strip (1412) and a third portion (1414c) of the support strip (1412) and wherein the support strip (1412) is wider at the first edge reinforcing region (1614) of the support strip (1412) than in the first portion (1414a) of the support strip (1412) and a third portion (1414c) of the support strip (1412);
the support strip (1412) is folded widthwise at the first edge reinforcing region (1614) to create a first edge fold region;
at a second edge reinforcing region of a support strip (1412), the support strip is folded over itself to create a double layer, wherein the second edge reinforcing region (1614) is positioned between a second portion (1414b) of the support strip (1412) and a fourth portion (1414d) of the support strip (1412) and wherein the support strip (1412) is wider at the second edge reinforcing region (1614) of the support strip (1412) than in the second portion (1414b) of the support strip (1412) and a fourth portion (1414c) of the support strip (1412);
the support strip (1412) is folded widthwise at the second edge reinforcing region (1614) to create a second edge fold region;
an end portion (1414e) of the third portion (1414c) of the support strip is coupled to an end portion (1414f) of the fourth portion (1414d) of the support strip to form a reinforced region of the support strip;
first and second outer reinforcing elements (1424a, 1424b) are positioned adjacent respective outer surfaces of the commissure tabs in the commissure tab pair;
the first and second outer reinforcing elements (1424a, 1424b) being coupled to the first and second commissure tabs (1430a, 1430b), respectively, via stitching (1464) that extends through: the first outer reinforcing element (1424a), the first commissure tab (1430a), the folded support strip (1412), the second commissure tab (1430b), and the second outer reinforcing element (1424b), the stitching extending over the reinforced region of the support strip; and
wherein, for each commissure, the support strip (1412) is secured to a respective commissure support portion (1450) of the plurality of commissure support portions.

11. The prosthetic valve of claim 10, wherein:
a) at the first edge reinforcing region of a support strip (1412), a first end portion and a second end portion of the first edge reinforcing region (1614) of the support strip (1412) are folded towards one another, and wherein, in the first end portion of the first edge reinforcing region (1614) prior to folding of the first end portion, a height of the support strip (1412) in the axial direction of the valve in which the support strip is included is increased on an upper region of the support strip compared to the height of the support strip (1412) in the first portion (1414a) and the third portion (1414c), and wherein, in the second end portion of the first edge reinforcing region (1614) prior to folding of the second end portion, a height of the support strip (1412) in the axial direction of the valve in which the support strip is included is increased on a lower region of the support strip compared to the height of the support strip (1412) in the first portion (1414a) and the third portion (1414c); and/or
b) at the second edge reinforcing region of the support strip (1412) a first end portion and a second end portion of the second edge reinforcing region (1614) of the support strip (1412) are folded towards one another, and wherein, in the first end portion of the second edge reinforcing region (1614) prior to folding of the first end portion, a height of the support strip (1412) in the axial direction of the valve in which the support strip is included is increased on the upper region of the support strip compared to the height of the support strip (1412) in the second portion (1414b) and the fourth portion (1414d), and wherein, in the second end portion of the second edge reinforcing region (1614) prior to folding of the second end portion, a height of the support strip (1412) in the axial direction of the valve in which the support strip is included is increased on the lower region of the support strip compared to the height of the support strip (1412) in the second portion (1414b) and the fourth portion (1414d);

12. The prosthetic heart valve of claim 10 or claim 11, wherein the first and the second edge reinforcing regions (1614) each include a plurality of stitching lines (1616), optionally wherein the stitching lines comprise:
a) an external marking visually indicating suturing locations for a user assembling the commissure; and/or
b) a plurality of apertures or openings through which a needle carrying sutures can pass.

13. The prosthetic heart valve of claim 12 insofar as it depends on claim 11 wherein, for each end portion of each edge reinforcing region (1614), one or more external markings, apertures and/or openings in an outer row of the respective end portion of the edge reinforcing region are aligned with one or more corresponding external markings, apertures and/or openings in an inner row of the respective end portion of the edge reinforcing region, such that each end portion at least partially overlaps itself when in the folded state.

14. The prosthetic heart valve of any of claims 10 to 13, wherein:
a) the reinforced region is positioned at substantially a vertical centerline of the support strip; and/or
b) the end portions (1414e, 1414f) of the third and fourth portions (1414c, 1414d) of the support strip are folded over stitches (1468) used for coupling the end portions to one another.

15. The prosthetic heart valve of any of claims 10 to 14, wherein the support strip is secured to the respective support portion of the frame via respective sutures (1466) stitched to folded portions of the support strip that are folded at the first and second edge fold regions, respectively, wherein the respective sutures are extended around the support portion of the frame and are attached to one another.
